# EUROPEAN PATENT APPLICATION

(11) **EP 4 818 314 A1**
(43) Date of publication of application: **07.10.2026**
(21) Application number: 25745486.8
(22) Date of filing: 21.01.2025
(51) Int. Cl.: C12N 9/00, C12N 15/77, C12P 19/32

(54) **NOVEL GLUTAMINE-HYDROLYZING GMP SYNTHASE VARIANT AND METHOD FOR PRODUCING 5'-GUANOSINE MONOPHOSPHATE BY USING SAME**

(30) Priority: 22.01.2024 KR 20240009487
(71) Applicant: CJ Cheiljedang Corporation, Seoul 04560 (KR)
(72) Inventor: KIM, Dae Young, Seoul 04560 (KR); KIM, Doyeon, Seoul 04560 (KR); LEE, Ji Hyun, Seoul 04560 (KR)
(74) Representative: Meissner Bolte Partnerschaft mbB
(86) International application number: PCT/KR2025/001134
(87) International publication number: WO 2025/159469

(57) **Abstract**

The present application relates to a novel glutamine-hydrolyzing GMP synthase variant and a method for producing 5'-guanosine monophosphate by using same. A microorganism into which the glutamine-hydrolyzing GMP synthase variant of the present application is introduced is cultured to enable high-yield GMP production.

## Description

### [TECHNICAL FIELD]

### Cross-reference to related application(s)

The present disclosure claims the benefit of priority based on Korean patent application No. 10-2024-0009487 filed on January 22, 2024, and the entire contents described in the documents of the corresponding Korean patent application are incorporated as part of this specification.

The present disclosure relates to a novel glutamine-hydrolyzing GMP synthase variant and a method for producing 5'-guanosine monophosphate using the same.

### [BACKGROUND ART]

5'-guanosine monophosphate (hereinafter, GMP) is an intermediate in the nucleic acid biosynthesis metabolic system, and has not only a physiologically important meaning in the bodies of animals and plants, but is also used for food, medicine, and various kinds of medical uses in many fields. In particular, it is one of nucleic acid-based seasonings that is spotlighted as savory seasonings, since it has a synergistic effect of taste when used with monosodium glutamate (MSG).

The method for producing GMP includes (1) a method for decomposing yeast RNA using microbial enzyme or chemically, (2) a method for directly producing nucleotides through a microorganism in a medium containing sugar and nitrogen sources, and phosphate sources, and (3) a method for chemically or enzymatically converting an intermediate of nucleotide synthesis. Currently, a combined producing method of fermentation and chemical synthesis and enzymatic conversion is widely used industrially.

This combined producing method consists of a fermentation process that produces 5'-xanthosine monophosphate (hereinafter, XMP), an intermediate product of the purine nucleotide biosynthesis metabolic system, and an enzymatic reaction process that converts the fermentation products into GMP, and microorganisms producing XMP and microorganisms capable of converting XMP into GMP are used. Therefore, for efficient GMP production, microorganisms involved in GMP production should inhibit GMP decomposition, and in particular, microorganisms added in the enzymatic reaction process should have a high complex ability to convert XMP into GMP.

For this reason, various studies are being conducted to develop high-efficiency production microorganisms and fermentation process technologies. For example, target substance-specific approach methods such as increasing expression of a gene encoding an enzyme involved in XMP or GMP biosynthesis or removing a gene unnecessary for biosynthesis are being mainly used (US 2020-0347346 A1).

### [DISCLOSURE]

### [TECHNICAL PROBLEM]

One embodiment of the present disclosure provides a polypeptide having glutamine-hydrolyzing GMP synthase activity.

The polypeptide may comprise an amino acid sequence in which, in the amino acid sequence having a sequence identity of 90% or more to the amino acid sequence of SEQ ID NO: 1, i) the amino acid corresponding to the 123rd residue of the amino acid sequence of SEQ ID NO: 1 is substituted with another amino acid, ii) the amino acid corresponding to the 441st residue of the amino acid sequence of SEQ ID NO: 1 is substituted with another residue, or iii) a combination thereof.

Another embodiment of the present disclosure provides a polynucleotide encoding the polypeptide.

Other embodiment of the present disclosure provides a recombinant vector comprising the polynucleotide.

Other embodiment of the present disclosure provides a microorganism, comprising at least one selected from the group consisting of the polypeptide having glutamine-hydrolyzing GMP synthase activity, a polynucleotide encoding the polypeptide and a vector comprising the polynucleotide.

Other embodiment of the present disclosure provides a method for producing GMP, comprising culturing the microorganism in a medium.

Other embodiment of the present disclosure provides a composition for producing 5'-guanosine monophosphate comprising the microorganism.

Other embodiment of the present disclosure provides a use of the microorganism for using in production of purine 5'-guanosine monophosphate.

Other embodiment of the present disclosure provides a use of the microorganism for using in production of a composition for producing 5'-guanosine monophosphate.

### [TECHNICAL SOLUTION]

In the present disclosure, a microorganism with excellent production capacity (conversion efficiency) of 5'-guanosine monophosphate (hereinafter, GMP) is to be provided by searching variants that enhance activity of glutamine-hydrolyzing GMP synthase, and introducing it into a microorganism or constructing microorganism comprising the variants.

In the present disclosure, it was confirmed that the production capacity (conversion efficiency) of 5'-guanosine monophosphate (GMP) was further increased, when an amino acid mutation was introduced into a specific position of glutamine-hydrolyzing GMP synthase.

One embodiment of the present disclosure provides a polypeptide having glutamine-hydrolyzing GMP synthase activity. The polypeptide may be a variant of glutamine-hydrolyzing GMP synthase derived from a microorganism of the genus *Corynebacterium*, and it may be a variant that enhances activity of glutamine-hydrolyzing GMP synthase.

In one embodiment, the polypeptide may comprise an amino acid sequence in which, from the N-terminus in the amino acid sequence having a sequence identity of 90% or more to the amino acid sequence of SEQ ID NO: 1, the amino acid corresponding to the 123rd residue of the amino acid sequence of SEQ ID NO: 1 is substituted with another amino acid, or the amino acid corresponding to the 441st residue is substituted with another amino acid, or a combination thereof.

In another embodiment, the polypeptide may comprise an amino acid sequence in which, from the N-terminus in the amino acid sequence having a sequence identity of 90% or more to the amino acid sequence of SEQ ID NO: 96, the amino acid corresponding to the 123rd residue of the amino acid sequence of SEQ ID NO: 96 is substituted with another amino acid, or the amino acid corresponding to the 441st residue is substituted with another amino acid, or a combination thereof.

The amino acid corresponding to the 123rd residue of the amino acid sequence of SEQ ID NO: 1, from the N-terminus in the amino acid sequence having a sequence identity of 90% or more to the amino acid sequence of SEQ ID NO: 1 may be the amino acid corresponding to the 123rd residue of the amino acid sequence of SEQ ID NO: 96, from the N-terminus in the amino acid sequence having a sequence identity of 90% or more to the amino acid sequence of SEQ ID NO: 96, and the amino acid corresponding to the 441st residue of the amino acid sequence of SEQ ID NO: 1, from the N-terminus in the amino acid sequence having a sequence identity of 90% or more to the amino acid sequence of SEQ ID NO: 1 may be the amino acid sequence corresponding to the 441st residue of the amino acid sequence of SEQ ID NO: 96, from the N-terminus in the amino acid sequence having a sequence identity of 90% or more to the amino acid sequence of SEQ ID NO: 96.

Counting amino acids from the N-terminus in the amino acid sequence as above, may mean counting with methionine (Met, M) translated from the start codon as the first amino acid.

Another embodiment of the present disclosure provides a polynucleotide encoding the polypeptide.

Other embodiment of the present disclosure provides a recombinant vector comprising the polynucleotide. The recombinant vector may be used as an expression vector of the polypeptide.

Other embodiment of the present disclosure provides a microorganism with enhanced activity of glutamine-hydrolyzing GMP synthase. The microorganism may be a microorganism producing GMP.

The microorganism with enhanced activity of glutamine-hydrolyzing GMP synthase may have high GMP production capacity, compared to a homogeneous microorganism in which glutamine-hydrolyzing GMP synthase is not enhanced.

Other embodiment of the present disclosure provides a method for producing GMP, comprising culturing the microorganism with enhanced activity of glutamine-hydrolyzing GMP synthase in a medium.

Other embodiment of the present disclosure provides a composition for producing GMP, comprising the microorganism with enhanced activity of glutamine-hydrolyzing GMP synthase.

Hereinafter, it will be described in more detail.

In the present description, the term, "glutamine-hydrolyzing GMP synthase" is an enzyme involved in converting 5'-xanthosine monophosphate (XMP) into 5'-guanosine monophosphate (hereinafter, GMP), and means an enzyme with activity that catalyzes the chemical reaction of ATP + H₂O + L-glutamine + XMP → AMP + diphosphate + GMP + 2H+ + L-glutamate. For the purpose of the present disclosure, the enzyme is a protein involved in producing 5'-guanosine monophosphate (hereinafter, GMP). Specifically, the glutamine-hydrolyzing GMP synthase of the present disclosure may be interchangeably used with "GMP synthesizing enzyme", "GMP synthase", "GMP synthetase", "5'-guanosine monophosphate biosynthetase", "GuaA protein". In the present disclosure, the sequence of the glutamine-hydrolyzing GMP synthase may be obtained in a known database, GenBank of NCBI (for example, WP_194285183.1 or WP_025387107.1).

The protein subject to mutation introduction of the present disclosure may be a wild-type protein with activity of glutamine-hydrolyzing GMP synthase. Specifically, the glutamine-hydrolyzing GMP synthase subject to mutation introduction may have or comprise the amino acid sequence of SEQ ID NO: 1 or SEQ ID NO: 96, or consist of the amino acid sequence, or be essentially consisting of the amino acid sequence, but not limited thereto. In other words, insignificant sequence addition before or after the amino acid sequence of SEQ ID NO: 1 or SEQ ID NO: 96, or mutations that may occur naturally, or silent mutations thereof are not excluded, and when it has activity identical or corresponding to a protein comprising the amino acid sequence of SEQ ID NO: 1 or SEQ ID NO: 96, it may correspond to a protein subject to mutation introduction of the present disclosure. For example, the protein subject to be mutation introduction of the present disclosure may be a protein consisting of an amino acid sequence with sequence homology or identity of 80%, 85%, 90%, 95%, 96%, 97%, 98%, 99%, 99.1%, 99.3%, 99.5%, 99.7%, or 99.9% or more, less than 100% to the amino acid sequence of SEQ ID NO: 1 or SEQ ID NO: 96. In addition, as long as it is an amino acid sequence which has such homology or identity and shows efficacy corresponding to the protein, proteins having an amino acid sequence in which some sequences are deleted, modified, substituted or added may also be comprised within the range of the protein subject to mutation of the present disclosure.

In the present disclosure, glutamine-hydrolyzing GMP synthase may be derived from a microorganism of the genus *Corynebacterium*, specifically, derived from *Corynebacterium stationis* (*Corynebacterium ammoniagenes*) or *Corynebacterium casei*, but not limited thereto.

One embodiment of the present disclosure provides a polypeptide having activity of glutamine-hydrolyzing GMP synthase comprising a mutation at the position corresponding to 123rd position and/or 441st position of the amino acid sequence of SEQ ID NO: 1, from the N-terminus in the amino acid sequence having a sequence identity of 90% or more to the amino acid sequence of SEQ ID NO: 1. The polypeptide may be a variant of glutamine-hydrolyzing GMP synthase. The variant of glutamine-hydrolyzing GMP synthase may increase activity of glutamine-hydrolyzing GMP synthase and/or GMP production capacity (conversion efficiency). The variant of glutamine-hydrolyzing GMP synthase may refer to a variant in which the amino acid corresponding to 123rd amino acid and/or 441st amino acid of the amino acid sequence of SEQ ID NO: 1, from the N-terminus in the amino acid sequence of SEQ ID NO: 1 described above and/or the amino acid having homology or identity of at least 80%, 85%, 90%, 95%, 96%, 97%, 98%, or 99% or more to SEQ ID NO: 1, is/are mutated.

As one example, the polypeptide having glutamine-hydrolyzing GMP synthase activity may consist of a polypeptide comprising an amino acid sequence in which, from the N-terminus in the amino acid sequence having a sequence identity of 90% or more to the amino acid sequence of SEQ ID NO: 1, i) the amino acid corresponding to the 123rd residue of the amino acid sequence of SEQ ID NO: 1 is substituted with another amino acid, ii) the amino acid corresponding to the 441st residue of the amino acid sequence of SEQ ID NO: 1 is substituted with another residue, or the amino acid corresponding to the 123rd residue of the amino acid sequence of SEQ ID NO: 1 is substituted with another amino acid, and the amino acid corresponding to the 441st residue of the amino acid sequence of SEQ ID NO: 1 is substituted with another amino acid. Another amino acid above may mean an amino acid other than the original amino acid.

Another embodiment of the present disclosure provides a polypeptide having glutamine-hydrolyzing GMP synthase activity comprising a mutation at the position corresponding to 123rd position and/or 441st position of the amino acid sequence of SEQ ID NO: 96, from the N-terminus in the amino acid sequence having a sequence identity of 90% or more to the amino acid sequence of SEQ ID NO: 96. The polypeptide may be a variant of glutamine-hydrolyzing GMP synthase. The variant of glutamine-hydrolyzing GMP synthase may increase activity of glutamine-hydrolyzing GMP synthase and/or increase GMP production capacity (conversion efficiency). The variant of glutamine-hydrolyzing GMP synthase may refer to a variant in which, the amino acid corresponding to 123rd amino acid and/or the 441st amino acid of the amino acid sequence of SEQ ID NO: 96, from the N-terminus in the amino acid sequence of SEQ ID NO: 96 described above, and/or the amino acid having homology or identity of at least 80%, 85%, 90%, 95%, 96%, 97%, 98%, or 99% or more to SEQ ID NO: 96, is/are mutated.

As one example, the polypeptide having glutamine-hydrolyzing GMP synthase activity may consist of a polypeptide comprising an amino acid sequence in which, from the N-terminus in the amino acid sequence having a sequence identity of 90% or more to the amino acid sequence of SEQ ID NO: 96, i) the amino acid corresponding to the 123rd residue of the amino acid sequence of SEQ ID NO: 96 is substituted with another amino acid, ii) the amino acid corresponding to the 441st residue of the amino acid sequence of SEQ ID NO: 96 is substituted with another amino acid, or iii) the amino acid corresponding to the 123rd residue of the amino acid sequence of SEQ ID NO: 96 is substituted with another amino acid, and the amino acid corresponding to the 441st residue of the amino acid sequence of SEQ ID NO: 96 is substituted with another amino acid. Another amino acid above may mean an amino acid other than the original amino acid.

In one embodiment, in the polypeptide having glutamine-hydrolyzing GMP synthase activity, the amino acid corresponding to the 123rd residue (position) of the amino acid sequence of SEQ ID NO: 1 or SEQ ID NO: 96 may be substituted with another amino acid. Another amino acid above may refer to an amino acid which is different from the original amino acid, aspartic acid, and specifically, it may be any one amino acid selected from the group consisting of lysine, alanine, cysteine, glutamic acid, phenylalanine, glycine, histidine, isoleucine, leucine, methionine, asparagine, proline, glutamine, arginine, serine, threonine, valine, tryptophan and tyrosine, and specifically, it may be any one amino acid selected from the group consisting of lysine, glutamic acid, histidine, methionine, glutamine, arginine, serine and tyrosine. In one specific embodiment, the polypeptide in which, in the amino acid sequence having a sequence identity of 90% or more to the amino acid sequence of SEQ ID NO: 1 or the amino acid sequence having a sequence identity of 90% or more to the amino acid sequence SEQ ID NO: 96, the amino acid corresponding to the 123rd residue of the amino acid sequence of SEQ ID NO: 1 or SEQ ID NO: 96 is substituted with another amino acid may comprise any one amino acid sequence selected from the group consisting of SEQ ID NO: 52 to SEQ ID NO: 59 and SEQ ID NO: 109, or consist of the amino acid sequence.

In one embodiment, in the polypeptide having glutamine-hydrolyzing GMP synthase activity, the amino acid corresponding to the 441st residue (position) of the amino acid sequence of SEQ ID NO: 1 or SEQ ID NO: 96 is substituted with another amino acid. Another amino acid above may refer to an amino acid which is different from the original amino acid, aspartic acid, and specifically, it may be any one amino acid selected from the group consisting of valine, alanine, cysteine, glutamic acid, phenylalanine, glycine, histidine, isoleucine, lysine, leucine, methionine, asparagine, proline, glutamine, arginine, serine, threonine, tryptophan and tyrosine, and specifically, it may be any one amino acid selected from the group consisting of valine, alanine, cysteine, glutamic acid, phenylalanine, histidine, isoleucine, lysine, leucine, asparagine, glutamine, threonine, and tryptophan. In one specific embodiment, the polypeptide in which the amino acid corresponding to the 441st residue in the amino acid sequence of SEQ ID NO: 1 or SEQ ID NO: 96 is substituted with another amino acid may comprise any one amino acid sequence selected from the group consisting of SEQ ID NO: 60 to SEQ ID NO: 72 and SEQ ID NO: 110, or consist of the amino acid sequence.

In one embodiment, in the polypeptide having glutamine-hydrolyzing GMP synthase activity, the amino acid corresponding to the 123rd residue of the amino acid sequence of SEQ ID NO: 1 or SEQ ID NO: 96 may be substituted with another amino acid, and the amino acid corresponding to the 441st residue of the amino acid sequence of SEQ ID NO: 1 or SEQ ID NO: 96 may be substituted with another amino acid. Another amino acid above may refer to an amino acid which is different from the original amino acid, and specifically, the amino acid corresponding to the 123rd residue may be substituted with lysine and the amino acid corresponding to the 441st residue may be substituted with valine. In one specific embodiment, the polypeptide in which the amino acid corresponding to the 123rd residue of the amino acid sequence of SEQ ID NO: 1 is substituted with another amino acid, and the amino acid corresponding to the 441st residue of the amino acid sequence of SEQ ID NO: 1 is substituted with another amino acid may comprise the amino acid sequence of SEQ ID NO: 73, or consist of the amino acid sequence.

In addition, the polypeptide having glutamine-hydrolyzing GMP synthase activity of the present disclosure may comprise a polypeptide having homology or identity of at least 80%, 85%, 90%, 95%, 96%, 97%, 98%, or 99% or more to the amino acid sequence in which the 123rd amino acid is substituted with another amino acid and/or the 441st amino acid is substituted with another amino acid from the N-terminus in the amino acid sequence of SEQ ID NO: 1 or SEQ ID NO: 96. In addition, it is obvious that as long as it is an amino acid sequence which has such homology or identity, and shows activity corresponding to the protein, proteins having an amino acid sequence in which some sequences are deleted, modified, substituted or added may be included within the range of the present disclosure.

In one specific embodiment, the polypeptide having glutamine-hydrolyzing GMP synthase activity may comprise an amino acid sequence having homology or identity of 70% or more, 75% or more, 80% or more, 85% or more, 90% or more, 95% or more, 96% or more, 96.3% or more, 96.5% or more, 96.7% or more, 96.9% or more, 97% or more, 97.1% or more, 97.2% or more, 97.4% or more, 97.6% or more, 97.8% or more, 98% or more, 98.2% or more, 98.4% or more, 98.6% or more, 98.9% or more, 99% or more, 99.1% or more, 99.3% or more, 99.5% or more, 99.7% or 99.9% or more to the amino acid sequence of any one SEQ ID NO selected from the group consisting of SEQ ID NOs: 52 to 73, or consist of the amino acid sequence.

In one specific embodiment, the polypeptide having glutamine-hydrolyzing GMP synthase activity may comprise an amino acid sequence having homology or identity of 70% or more, 75% or more, 80% or more, 85% or more, 90% or more, 95% or more, 96% or more, 96.3% or more, 96.5% or more, 96.7% or more, 96.9% or more, 97% or more, 97.1% or more, 97.2% or more, 97.4% or more, 97.6% or more, 97.8% or more, 98% or more, 98.2% or more, 98.4% or more, 98.6% or more, 98.9% or more, 99% or more, 99.1% or more, 99.3% or more, 99.5% or more, 99.7% or 99.9% or more to the amino acid sequence of any one SEQ ID NO selected from the group consisting of SEQ ID NOs: 106 to 108, or consist of the amino acid sequence.

In addition, as long as it is a polypeptide showing activity corresponding to the polypeptide having homology or identity and having the glutamine-hydrolyzing GMP synthase activity, even if it has an amino acid sequence in which some sequences are deleted, modified, substituted, conservatively substituted and/or added, it may be comprised in the variant of the present disclosure. For example, it may be a case having sequence addition or deletion, mutation that may occur naturally, silent mutation, or conservative substitution, which does not alter the activity of the variant at the N-terminus, C-terminus and/or inside of the amino acid sequence of the variant of glutamine-hydrolyzing GMP synthase of the present disclosure.

The "conservative substitution" means substituting one amino acid with another amino acid having similar structural and/or chemical properties. This amino acid substitution may occur generally based on the similarity in polarity, charge, solubility, hydrophobicity, hydrophilicity and/or amphipathic nature of residues. In common, conservative substitution may hardly affect or not affect activity of protein or polypeptide.

In one embodiment, in the amino acid sequence having a sequence identity of 90% or more to the amino acid sequence of SEQ ID NO: 1, the amino acid corresponding to the 123rd residue of the amino acid sequence of SEQ ID NO: 1 and/or the amino acid corresponding to the 441st residue may be aspartic acid (Aspartate, Asp, D), but not limited thereto.

In one embodiment, in the amino acid sequence having a sequence identity of 90% or more to the amino acid sequence of SEQ ID NO: 96, the amino acid corresponding to the 123rd residue of the amino acid sequence of SEQ ID NO: 96 may be aspartic acid (Aspartate, Asp, D), and the amino acid corresponding to the 441st residue may be glutamic acid (Glutamate, Glu, E), but not limited thereto.

The polypeptide having glutamine-hydrolyzing GMP synthase activity of the present disclosure may have a characteristic of increasing GMP production capacity (conversion efficiency) compared to a wild-type polypeptide having activity of glutamine-hydrolyzing GMP synthase.

In the present disclosure, the term, "GMP (5'-guanosine monophosphate; hereinafter, GMP)" is one type of nucleotides used as a unit in RNA and refers to an intermediate of the nucleic acid biosynthesis metabolic system. The "GMP" may be interchangeably used with "5'-guanosine monophosphate", and may be synthesized by adding an ammonia molecule to XMP by glutamine-hydrolyzing GMP synthase. The method for preparing GMP from XMP and/or means used for the method may be selected from known technologies.

In one embodiment, the GMP may be prepared by being converted from XMP, but not limited thereto.

In the present disclosure, the term, "variant" refers to a polypeptide which is different from the amino acid sequence before modification of the variant, as at least one amino acid is conservatively substituted and/or modified, but maintains functions or properties. Such a variant may be generally identified by modifying at least one amino acid in the amino acid sequence of the polypeptide and evaluating properties of the modified polypeptide. In other words, the ability of the variant may be increased, not changed, or reduced, compared to the polypeptide before modification. In addition, some variants may include variants in which at least one part such as a N-terminal leader sequence or transmembrane domain is removed. Other variants may include variants in which a part is removed from the N- and/or C-terminus of mature protein. The term, "variant" may be interchangeably used with terms of mutant, modified, mutant polypeptide, mutated protein, mutation, and variant and the like (in English expressions, modification, modified polypeptide, modified protein, mutant, mutein, divergent, variant, etc.), and as long as it is a term used for a mutated meaning, it is not limited thereto. For the purpose of the present disclosure, the variant may be a polypeptide having glutamine-hydrolyzing GMP synthase activity, and comprising an amino acid sequence in which, in the amino acid sequence having a sequence identity of 90% or more to the amino acid sequence of SEQ ID NO: 1 or having a sequence identity of 90% or more to the amino acid sequence SEQ ID NO: 96, the amino acid corresponding to the 123rd residue of the amino acid sequence of SEQ ID NO: 1 or SEQ ID NO: 96 is substituted with another amino acid, or the amino acid corresponding to the 441st residue is substituted with another amino acid, or a combination thereof.

Furthermore, the variant may comprise deletion or addition of amino acids having minimal effects on the properties and secondary structure of the polypeptide. For example, a signal (or leader) sequence involved in protein translocation co-translationally or post-translationally may be conjugated at the N-terminus of the variant. In addition, the variant may be conjugated with another sequence or linker to be identified, purified, or synthesized.

In one embodiment, the variant may be encoded by a polynucleotide comprising any one nucleic acid sequence selected from SEQ ID NO: 74 to SEQ ID NO: 95 and SEQ ID NOs: 109 to 111/ or consisting of the nucleic acid sequence.

Other embodiment of the present disclosure provides a polynucleotide encoding the polypeptide having glutamine-hydrolyzing GMP synthase activity (for example, variant of glutamine-hydrolyzing GMP synthase).

In the present disclosure, the term, "polynucleotide" means a DNA or RNA strand of a certain length or longer, which is a polymer of nucleotides in which nucleotide monomers are linked together in a long chain shape by covalent bonds.

A polynucleotide encoding the polypeptide having glutamine-hydrolyzing GMP synthase activity (for example, variant of glutamine-hydrolyzing GMP synthase) of the present disclosure may be included without limitation, as long as it is a polynucleotide sequence encoding a polypeptide having glutamine-hydrolyzing GMP synthase activity. In the present disclosure, the gene encoding the amino acid sequence of glutamine-hydrolyzing GMP synthase is guaA gene, and may be derived from a microorganism of the genus *Corynebacterium*, and specifically, it may be derived from *Corynebacterium stationis* or *Corynebacterium casei*, but not limited thereto.

In one embodiment, the polynucleotide may comprise a nucleic acid sequence (base sequence) described as any one SEQ ID NO selected from SEQ ID NO: 74 to SEQ ID NO: 95 and SEQ ID NOs: 109 to 111, or consist of or be essentially consisting of a nucleic acid sequence of any one SEQ ID NO selected from SEQ ID NO: 74 to SEQ ID NO: 95 and SEQ ID NOs: 109 to 111.

The polynucleotide consisting of any one nucleic acid sequence selected from SEQ ID NO: 74 to SEQ ID NOs: 95 and SEQ ID NO: 109 to 111 or comprising the sequence may encode an amino acid sequence described as any one SEQ ID NO selected from SEQ ID NO: 52 to 73 and SEQ ID NOs: 106 to 108, respectively.

The polynucleotide of the present disclosure may have various modifications made to the coding region within a range that does not change the amino acid sequence of the variant of the present disclosure, in consideration of codon degeneracy or codons preferred in the organism in which the variant of the present disclosure is to be expressed. Specifically, the polynucleotide of the present disclosure may have or comprise a base sequence having homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to any one base sequence selected from SEQ ID NO: 74 to SEQ ID NO: 95 and SEQ ID NOs: 109 to 111, or may consist of or be essentially consisting of a base sequence with homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more to any one sequence selected from SEQ ID NO: 74 to SEQ ID NO: 95 and SEQ ID NOs: 109 to 111, but not limited thereto.

The polynucleotide of the present disclosure may comprise without limitation, a probe that may be prepared from a known genetic sequence, for example, a sequence that may be hybridized under stringent conditions with a sequence complementary to all or a part of the polynucleotide sequence of the present disclosure. The "stringent condition" means a condition that enables specific hybridization between polynucleotides. This condition is specifically described in the document (See J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York, 9.50-9.51, 11.7-11.8). For example, a condition of hybridizing polynucleotides with high homology or identity, polynucleotides with homology or identity of 70% or more, 75% or more, 80% or more, 85% or more, 90% or more, 95% or more, 96% or more, 97% or more, 98% or more, 99% or more, 99.1% or more, 99.2% or more, 99.3% or more, 99.4% or more, 99.5% or more, 99.6% or more, 99.7% or more, 99.8% or more, or 99.9% or more, and not hybridizing polynucleotides with homology or identity lower than that, or a washing condition of common southern hybridization, a condition of washing at a salt concentration and temperature corresponding to 60°C, 1×SSC, 0.1% SDS, specifically, 60°C, 0.1×SSC, 0.1% SDS, more specifically, 68°C, 0.1×SSC, 0.1% SDS, once, specifically, twice to three times may be listed.

Hybridization requires that two nucleotides be complementary sequences, but hybridized polynucleotides may comprise some mismatches between bases depending on the stringency of hybridization. The term, "complementary" is used to describe the relationship between nucleotide bases that may hybridize with each other. For example, with respect to DNA, adenine is complementary to thymine, and cytosine is complementary to guanine. Therefore, the polynucleotide of the present disclosure may also comprise isolated nucleic acid fragments complementary to the entire sequence as well as substantially similar nucleic acid sequences.

Specifically, the polynucleotide having homology or identity to the polynucleotide of the present disclosure may be detected using the hybridization conditions comprising a hybridization step at a Tm value of 55 °C and using the aforementioned conditions. In addition, the Tm value may be 60 °C, 63 °C or 65 °C, but not limited thereto, and may be appropriately adjusted by those skilled in the art according to its purpose.

The appropriate stringency that hybridizes the polynucleotide depends on the length of the polynucleotide and degree of complementarity and variables are well known in the corresponding technical field (for example, J. Sambrook et al., same above).

In the present description, that a polynucleotide (may be interchangeably used with "gene") or polypeptide (may be interchangeably used with "protein") "comprises a specific nucleic acid sequence or amino acid sequence or consists of or is expressed as a specific nucleic acid sequence or amino acid sequence" may mean that the polynucleotide or polypeptide essentially comprises the specific nucleic acid sequence or amino acid sequence, and may be interpreted as comprising "a substantially equivalent sequence" in which an insignificant mutation (deletion, substitution, modification, and/or addition) is added to the specific nucleic acid sequence or amino acid sequence within the range of maintaining the original function and/or targeted function of the polynucleotide or polypeptide (or not excluding the insignificant mutation).

In the present description, the term, "homology" or "identity" means a degree of similarity between two given amino acid sequences or base sequences, and may be expressed as a percentage. The terms, homology and identity may be often used interchangeably.

The sequence homology or identity of the conserved polynucleotide or polypeptide may be determined by a standard array algorithm, and a default gap penalty established by a used program may be used together. Substantially, the homologous or identical sequence mya be generally hybridized with all or part of the sequence under moderate or high stringent conditions. It is obvious that hybridization includes hybridization with a polynucleotide containing a common codon or codon considering codon degeneracy in the polynucleotide.

Whether any two polynucleotide or polypeptide sequences have homology or identity, may be determined using a default parameter as in Pearson et al (1988) [Proc. Natl. Acad. Sci. USA 85]: 2444 using a known computer algorithm such as "FASTA" program. Otherwise, as performed in Needleman program of EMOSS package (EMBOSS: The European Molecular Biology Open Software Suite, Rice et al., 2000, Trends Genet. 16: 276-277) (version 5.0.0 or later version), it may be determined using Needleman-Wunsch algorithm (Needleman and Wunsch, 1970, J. Mol. Biol. 48: 443-453) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, using BLAST, or ClustalW of National Center of Biotechnology Information Database, the homology or identity may be determined.

The homology or identity of the polynucleotide or polypeptide may be determined by comparing sequence information using GAP computer program such as for example, Needleman et al. (1970), J Mol Biol. 48:443, as known in for example, Smith and Waterman, Adv. Appl. Math (1981) 2:482. In summary, GAP program may be defined as the total number of symbols in the shorter of two sequences divided by the number of similarly arranged symbols (i.e., nucleotides or amino acids). The default parameters for the GAP program may comprise (1) a binary comparison matrix (containing values of 1 for identity and 0 for non-identity) and a weighted comparison matrix of Gribskov et al (1986) Nucl. Acids Res. 14: 6745, disclosed in Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) a 3.0 penalty for each gap and an additional 0.10 penalty for each symbol in each gap (or gap opening penalty 10, gap extension penalty 0.5); and (3) no penalty for end gaps.

In the present description, the term, "corresponding to" refers an amino acid residue at the listed position in the polypeptide, or an amino acid residue similar, identical, or homologous to the listed residue in the polypeptide. Conforming the amino acid at the corresponding position may be determining a specific amino acid in a sequence that references a particular sequence. The "corresponding region" used in the present disclosure generally refers to a similar or corresponding position in a related protein or reference protein.

For example, any amino acid sequence may be aligned with SEQ ID NO: 1, and based on this, each amino acid residue of the amino acid sequence may be numbered by referring to the numerical position of the amino acid residue corresponding to the amino acid residue of SEQ ID NO: 1. For example, the sequence alignment algorithm as described in the present disclosure may identify positions of amino acids, or positions where modifications such as substitution, insertion or deletion or the like occur, by comparing with a query sequence (also referred to as a "reference sequence").

For this alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277) and the like may be used, but not limited thereto, and sequence alignment programs known in the art, pairwise sequence comparison algorithm and the like may be appropriately used.

Other embodiment of the present disclosure provides a vector comprising a polynucleotide encoding the polypeptide having glutamine-hydrolyzing GMP synthase activity (for example, variant of glutamine-hydrolyzing GMP synthase). The vector may be a vector for insertion or an expression vector.

In the present description, the term, "vector" means a DNA product for delivering a target polynucleotide in a suitable host or host cell. As one example, it may comprise a base sequence of a polynucleotide encoding a target polypeptide operably linked to a suitable expression regulatory region (or expression regulatory sequence) so as to express the target polypeptide in a suitable host cell, but is not limited thereto. The regulatory sequence may comprise a promoter which can initiate transcription, any operator sequence for regulating transcription, a sequence encoding a suitable mRNA ribosome binding site, and/or a sequence that regulates termination of transcription and/or translation. The vector may be maintained independently of genome of the host cell, or be inserted into genome of the host cell, after being transformed into an appropriate host cell. As one example, through a vector for insertion, the target polynucleotide may be inserted into chromosome. The insertion of the polynucleotide into chromosome may be achieved by any method known in the art, for example, homologous recombination, but is not limited thereto.

In the present description the available vector is not particularly limited as long as it is replicable in a host cell, and it may be selected from all commonly used vectors. The examples of the commonly used vectors may include plasmids, cosmids, viruses, bacteriophages, and the like in a natural state or recombinant state. For example, as the vector, as the phage vector or cosmid vector, pWE15, M13, MBL3, MBL4, IXII, ASHII, APII, t10, t11, Charon4A, and Charon21A and the like may be used, and as the plasmid vector, pBR-based, pUC-based, pBluescriptII-based, pGEM-based, pTZ-based, pCL-based and pET-based and the like may be used. Specifically, pDZ, pACYC177, pACYC184, pCL, pECCG117, pUC19, pBR322, pMW118, pCC1BAC, pDCM2, pDC24 vectors and the like may be exemplified, but not limited thereto.

The vector may further comprise a selection marker for confirming introduction into transformed cells or insertion of the transformed cells into genome. The selection marker is to confirm insertion of the cells transformed with the vector or the polynucleotide, and may be used as selected from genes that confer selectable phenotypes such as drug resistance, auxotrophy, resistance to a cytotoxic agent or expression of surface proteins. Under the environment treated with a selective agent, only cells expressing a selection marker are survived or show other phenotypes, so transformed cells may be selected.

Expressing the polypeptide (variant) in a microorganism may be performed by introducing a polynucleotide encoding the variant, or a vector comprising thereof into a host cell, and culturing a recombinant cell (for example, microorganism) comprising the same.

The introduction of a polynucleotide encoding the polypeptide (variant) or a vector comprising the same into a microorganism may be performed by appropriately selecting a known transformation method by those skilled in the art. In the present description, the term, "transformation" refers to introducing a target polynucleotide or a vector comprising the same into a shot cell (microorganism) to change genetic characteristics of the host cell (microorganism). The transformed polynucleotide may be inserted and positioned in chromosome of the host cell or be positioned outside the chromosome. The polynucleotide may be introduced in an appropriate form depending on the purpose of introduction. For example, the polynucleotide may be introduced into a host cell in an expression cassette form, which is a gene structure comprising all elements required for being expressed by itself. The expression cassette may commonly comprise expression regulatory elements of a promoter operably linked to the polynucleotide, a transcription termination signal, a ribosome binding site, and/or a translation termination signal and the like. The expression cassette may be in a form of an expression vector capable of self-replication. In addition, the polynucleotide may be introduced into a host cell in its own form and be operably linked to a sequence required for expression in the host cell. In the above, the term, "operably linked" may mean that expression regulatory elements (e.g., promoter) and a polynucleotide may be functionally linked so that transcriptional regulation (e.g., transcription initiation) of the polynucleotide can be performed. Operable linking may be performed using a genetic recombinant technology known in the art.

The method for transforming the polynucleotide into a host cell may be performed by any method for introducing nucleic acid into a cell (microorganism), and may be performed by appropriately selecting a transformation technology known in the art. As the known transformation method, electroporation, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cation liposome method, lipofection, lithium citrate-DMSO method, and the like may be exemplified, but is not limited thereto.

Other embodiment of the present disclosure provides a microorganism with enhanced glutamine-hydrolyzing GMP synthase activity.

The microorganism may be a microorganism comprising at least one (for example, one kind or more, 2 kinds or more, or 1 kind, 2 kinds, or 3 kinds) selected from the group consisting of the afore-mentioned polypeptide (variant) having glutamine-hydrolyzing GMP synthase activity, a polynucleotide encoding (or coding) the polypeptide, and a vector comprising the polynucleotide.

Other embodiment of the present disclosure provides a microorganism with enhanced activity.

The microorganism may be a microorganism comprising at least one (for example, one kind or more, 2 kinds or more, or 1 kind, 2 kinds, or 3 kinds) selected from the group consisting of the afore-mentioned polypeptide (variant) having activity, a polynucleotide encoding (or coding) the polypeptide, and a vector comprising the polynucleotide.

In the present description, the term, "enhancement" of the polypeptide activity (for example, glutamine-hydrolyzing GMP synthase activity) refers to increasing activity of the polypeptide in a host cell (microorganism) compared to the intrinsic activity. The enhancement may be interchangeably used with the terms such as activation, up-regulation, overexpression, increase and the like. Herein, the activation, enhancement, up-regulation, overexpression and increase may include showing activity that is not originally present, or exhibiting activity improved than intrinsic activity or activity before modification. The "intrinsic activity" means activity of a specific polypeptide which a parent strain before changing traits or non-modified microorganism has originally, when traits are changed by genetic mutation due to natural or artificial factors. This may be interchangeably used with "activity before modification". That the activity of the polypeptide is "enhanced", "up-regulated", "overexpressed" or "increased" compared to the intrinsic activity, means that it is improved compared to the activity and/or concentration (expression level) of a specific polypeptide that a parent strain before changing traits or non-modified microorganism has originally.

The enhancement may be achieved by introducing a foreign polypeptide, or enhancement of activity of an intrinsic polypeptide and/or concentration (expression level). Whether the activity of the polypeptide is enhanced may be confirmed by the degree of activity, expression level of the corresponding polypeptide, or an increase of the amount of products released from the corresponding polypeptide.

For the enhancement of the activity of the polypeptide, various methods well known in the art may be applied, and there is no limitation, as long as the activity of a target polypeptide can be enhanced compared to a microorganism before modification. Specifically, genetic engineering and/or protein engineering well known to those skilled in the art, which are routine methods of molecular biology, may be used, but not limited thereto (for example, Sitnicka et al. Functional Analysis of Genes. Advances in Cell Biology. 2010, Vol. 2. 1-16, Sambrook et al. Molecular Cloning 2012, etc.).

Specifically, the enhancement of the polypeptide of the present disclosure may be
1) an increase of a copy number in cells of a polynucleotide encoding a polypeptide;
2) replacement of a gene expression regulatory region on chromosome encoding a polypeptide with a sequence having strong activity;
3) modification of a base sequence encoding an initiation codon or 5'-UTR area of gene transcriptome encoding a polypeptide;
4) modification of an amino acid sequence of the polypeptide so that the activity of the polypeptide is enhanced;
5) modification of a polynucleotide sequence encoding the polypeptide so that the activity of the polypeptide is enhanced (for example, modification of a polynucleotide of the polypeptide gene so as to encode a polypeptide modified to enhance the activity of the polypeptide);
6) introduction of a foreign polypeptide showing activity of a polypeptide or a foreign polynucleotide encoding the same;
7) codon optimization of a polynucleotide encoding a polypeptide;
8) modification or chemical modification by analyzing the tertiary structure of a polypeptide and selecting an exposed site; or
9) adjustment of cellular localization of a protein (polypeptide); or
10) a combination of at least 2 selected from the 1) to 9), but not particularly limited thereto.

More specifically,
the 1) increase of a copy number in cells of a polynucleotide encoding a polypeptide may be achieved by introduction of a vector which can replicate and function regardless of a host, in which a polynucleotide encoding the corresponding polypeptide is operatively linked. Otherwise, it may be achieved by introduction of 1 copy or 2 copies or more of the polynucleotide encoding the corresponding polypeptide into chromosome in a host cell. The introduction into the chromosome may be performed by introducing a vector capable of inserting the polynucleotide into chromosome of the host cell into the host cell, but not limited thereto. The vector is as described above.

The 2) replacement of a gene expression regulatory region (or expression regulatory sequence) on chromosome encoding a polypeptide with a sequence having strong activity, may be for example, occurrence of mutation in the sequence by deletion, insertion, non-conservative or conservative substitution or a combination thereof so as to further intensify activity of the expression regulatory region, or replacement with a sequence having stronger activity. The expression regulatory region is not particularly limited thereto, but may comprise a promoter, an operator sequence, a sequence encoding a ribosome binding site, and a sequence regulating termination of transcription and translation and the like. As one example, the original promoter may be replaced with a strong promoter, but not limited thereto.

Examples of the known strong promoter include CJ1 to CJ7 promoters (U.S. Patent US 7662943 B2), lac promoter, trp promoter, trc promoter, tac promoter, lambda phage PR promoter, PL promoter, tet promoter, gapA promoter, SPL7 promoter, SPL13(sm3) promoter (U.S. Patent US 10584338 B2), O2 promoter (U.S. Patent US 10273491 B2), tkt promoter, yccA promoter, and the like, but not limited thereto.

The 3) modification of a base sequence encoding an initiation codon or 5'-UTR area of gene transcriptome encoding a polypeptide may be for example, substituting it with a base sequence encoding another initiation codon with a higher polypeptide expression rate compared to the intrinsic initiation codon, but not limited thereto.

The modification of an amino acid sequence or polynucleotide sequence of the 4) and 5) may be occurrence of mutation in the sequence by deletion, insertion, non-conservative or conservative substitution or a combination thereof, or replacement with an amino acid sequence or polynucleotide sequence improved to have stronger activity for the amino acid sequence of the polypeptide or a polynucleotide sequence encoding the polypeptide so as to strengthen the activity of the polypeptide, but not limited thereto. The replacement may be performed specifically by inserting a polynucleotide into chromosome by homologous recombination, but not limited thereto. The vector used then may further comprise a selection marker for confirming chromosome insertion. The selection marker is as described above.

The 6) introduction of a foreign polypeptide showing activity of a polypeptide may be introduction of a foreign polynucleotide encoding a polypeptide showing the same/similar activity to the polypeptide into a host cell. The foreign polynucleotide is not limited in its origin or sequence as long as it shows the same/similar activity to the polypeptide. The method used for the introduction may be performed by those skilled in the art by appropriately selecting a known transformation method, and by expressing the introduced polynucleotide in a host cell, a polypeptide may be produced and its activity may be increased.

The 7) codon optimization of a polynucleotide encoding a polypeptide may be codon optimization so that transcription or translation of an intrinsic polynucleotide is increased in a host cell, or codon optimization so that a foreign polynucleotide is optimized in a host cell to achieve transcription and translation.

The 8) modification or chemical modification by analyzing the tertiary structure of a polypeptide and selecting an exposed site may determine template protein candidates depending on the degree of similarity of sequences by comparing sequence information of a polypeptide to be analyzed, and confirm the structure based on this, and select an exposed site to be modified or chemically modified, and transform or modify it.

The 9) adjustment of cellular localization of a polypeptide may target a polypeptide into an intracellular specific organelle or specific intracellular space. For example, by addition or removal of a leader sequence functioning in targeting a polypeptide, it may target it to periplasm or cytoplasm, but not limited thereto.

Such enhancement of the polypeptide activity means increasing activity or concentration expression level of the corresponding polypeptide based on the activity or concentration of the expressed polypeptide in a wild-type or pre-transformed microbial strain, or increasing the amount of products produced from the corresponding polypeptide, but is not limited thereto.

In the present description, the term, "microorganism (or, strain)" may include all wild-type microorganisms or microorganisms in which genetic modification occurs naturally or artificially. The microorganism is a microorganism in which a specific mechanism is enhanced or weakened due to reasons such as insertion of an external gene or enhancement or weakening of activity of an endogenous gene, and may be a microorganism comprising genetic modification for producing a targeted polypeptide, protein or product (for example, GMP). In the present disclosure, the terms, "microorganism", "strain", "host" and "host cell" may be interchangeably used.

The microorganism of the present disclosure may be a microorganism having enhanced activity of glutamine-hydrolyzing GMP synthase, or having improved (or increased) GMP conversion efficiency, or having GMP production capacity (or production), or having improved (or increased) GMP production capacity.

In the present disclosure, "GMP production capacity" may be interchangeably used with "GMP conversion efficiency".

As one example, the microorganism of the present disclosure may be a microorganism in which GMP production capacity is given or improved, as the polypeptide (variant) having glutamine-hydrolyzing GMP synthase activity of the present disclosure or a polynucleotide encoding the same is introduced into a microorganism having no GMP production capacity (conversion efficiency), or a microorganism having GMP production capacity naturally, but not limited thereto.

In the present description, the term, "microorganism with enhanced glutamine-hydrolyzing GMP synthase activity" may mean that a microorganism having no GMP production ability has GMP production capacity, or has higher GMP production capacity than the original GMP production capacity, as engineered (mutated) to express the afore-mentioned polypeptide (variant) having glutamine-hydrolyzing GMP synthase activity.

In the present disclosure, "non-modified microorganism" does not exclude strains comprising a mutation that may occur naturally in a microorganism, and may mean a wild-type strain, or a natural strain itself, or a train before changing traits by genetic mutation due to natural or artificial factors. For example, the non-modified microorganism may mean a strain in which the polypeptide (variant) having glutamine-hydrolyzing GMP synthase activity of the present disclosure or a polynucleotide encoding the polypeptide (variant) having glutamine-hydrolyzing GMP synthase activity is not introduced, or before introduced according to one embodiment. The "non-modified microorganism" may be interchangeably used with "strain before modified", "microorganism before modified", "non-modified strain", "non-mutated microorganism" or "reference microorganism".

In the present disclosure, the reference microorganism may be a wild-type microorganism known to produce GMP, and for example, may be *Corynebacterium stationis* ATCC6872. Otherwise, the reference microorganism may be a microorganism known to produce GMP or convert XMP into GMP, and for example, may be *Corynebacterium stationis* KCCM13320P, but is not limited thereto. In addition, the reference microorganism may be wild-type *Corynebacterium casei*, and specifically, may be *Corynebacterium casei* LMG S-19264 strain, but is not limited thereto.

The microorganism producing GMP of the present disclosure is not particularly limited as long as it may produce GMP, but may be a microorganism of the genus *Corynebacterium*. The microorganism of the genus *Corynebacterium* may be at least one microorganism selected from the group consisting of *Corynebacterium stationis*, *Corynebacterium casei*, *Corynebacterium thermoaminogenes*, *Corynebacterium glutamicum*, *Brevibacterium flavum*, *Brevibacterium lactofermentum*, *Corynebacterium crudilactis*, *Corynebacterium deserti*, *Corynebacterium efficiens*, *Corynebacterium callunae*, *Corynebacterium singulare*, *Corynebacterium halotolerans*, *Corynebacterium striatum*, *Corynebacterium pollutisoli*, *Corynebacterium imitans*, *Corynebacterium testudinoris*, and *Corynebacterium flavescens*, but not limited thereto.

Other embodiment of the present disclosure provides a method for producing GMP, comprising; culturing a microorganism with enhanced activity of glutamine-hydrolyzing GMP synthase in a medium.

The microorganism with enhanced activity of glutamine-hydrolyzing GMP synthase and GMP are as described above.

In the present disclosure, "culturing" means growing a microorganism, for example, a microorganism of the genus *Corynebacterium*, in which the polypeptide having glutamine-hydrolyzing GMP synthase activity of the present disclosure or a gene encoding the same is introduced or its activity is enhanced, under appropriately controlled environmental conditions. The culturing process of the present disclosure may be conducted according to appropriate medium and culturing conditions known in the art. This culturing process may be easily adjusted and used by those skilled in the art depending on the selected strain. Specifically, the culturing may be batch, continuous, and/or fed-batch, but not limited thereto.

In the present disclosure, "medium" means a substance in which nutrients required for culturing a microorganism, for example, a microorganism of the genus *Corynebacterium*, in which the polypeptide having glutamine-hydrolyzing GMP synthase activity of the present disclosure or a gene encoding the same is introduced or its activity is enhanced, are mixed as main ingredients, and it supplies nutrients and growth factors including water essential for survival and development. In addition, XMP may be included in the medium for GMP synthesis. Specifically, the medium and other culture conditions used for culturing the microorganism of the present disclosure may be used without a particular limitation as long as it is a medium used for culturing a common microorganism, but the microorganism of the present disclosure may be cultured under aerobic conditions in a common medium containing an appropriate carbon source, a nitrogen source, a phosphorous source, an inorganic compound, an amino acid, and/or a vitamin and the like, while adjusting temperature, pH and the like.

In the present disclosure, the carbon source may include carbohydrates such as glucose, saccharose, lactose, fructose, sucrose, maltose, etc.; sugar alcohols such as mannitol, sorbitol, etc.; organic acids such as pyruvate, lactate, citrate, etc.; amino acids such as glutamic acid, methionine, lysine, etc., and the like. In addition, natural organic nutrients such as starch hydrolysate, molasses (for example, blackstrap molasses), rice bran, cassava, sugar cane residues and corn steep liquor may be used, and specifically, carbohydrates such as glucose and sterilized pre-treated molasses (i.e., molasses converted into reducing sugar) and the like may be used, and other appropriate carbon sources may be used variously without limitation. These carbon sources may be used alone or be used in combination of two or more kinds, but not limited thereto.

As the nitrogen source, inorganic nitrogen sources such as ammonia, ammonium sulfate, ammonium chloride, ammonium citrate, ammonium phosphate, ammonium carbonate, ammonium nitrate and the like, and organic nitrogen sources such as amino acids such as glutamic acid, methionine, glutamine and the like, peptone, NZ-amine, meat extract, yeast extract, malt extract, corn steep liquor, casein hydrolysate, fish or its decomposition product, defatted soybean cake or its decomposition product and the like may be used. These nitrogen sources may be used alone or be used in combination of two or more kinds, but not limited thereto.

The phosphorus source may include monopotassium phosphate, dipotassium phosphate, or sodium-containing salts corresponding thereto and the like. As the inorganic compound, sodium chloride, calcium chloride, iron chloride, magnesium sulfate, iron sulfate, manganese sulfate, calcium carbonate and the like may be used, and other amino acids, vitamins, and/or appropriate precursors and the like may be comprised. These components or precursors may be added to a medium in a batch or continuous manner. However, it is not limited thereto.

In addition, during culture of the microorganism of the present disclosure, a compound such as ammonium hydroxide, potassium hydroxide, ammonia, phosphate, sulfate, and the like is added to a medium by an appropriate method, to adjust the pH of the medium. Furthermore, during culture, using an antifoaming agent such as fatty acid polyglycol ester, air formation may be inhibited. In addition, in order to maintain the aerobic condition of the medium, oxygen or oxygen-containing gas may be injected into the medium, or in order to maintain the anaerobic and non-aerobic condition, without injection of gas, or nitrogen, hydrogen or carbon dioxide gas may be injected, but not limited thereto.

The culture temperature in the culture of the present disclosure may be maintained at 20 to 45°C, or 25 to 37°C, specifically, 25 to 37°C, and the culture may be performed for about 10 to 160 hours, or about 20 to 120 hours, but not limited thereto.

The GMP produced by culture of the present disclosure may be secreted into the medium or remain within the cells.

The method for producing GMP of the present disclosure may comprise adding an enzyme in a medium or adding a microorganism expressing the enzyme. For example, the method may further comprise adding an enzyme converting XMP into GMP or a microorganism expressing the enzyme and/or culturing the microorganism, after the culturing a microorganism producing XMP.

In one embodiment, the method for producing GMP of the present disclosure may further comprise the step of culturing a microorganism producing 5'-xanthic acid (XMP), or the step of adding XMP to the medium, prior to the step of culturing the microorganism with enhanced activity of glutamine-hydrolyzing GMP synthase in a medium.

The method for producing GMP of the present disclosure may further comprise recovering GMP from the cultured microorganism (for example, microorganism of the genus *Corynebacterium*), a medium of culture (medium in which culture is performed) or both of them. The recovering may be further comprised after the culturing.

The recovery may collect targeted purine nucleotides using an appropriate method known in the art according to the culture method of a microorganism of the present disclosure, for example, a batch, continuous, or fed-batch culture method. For example, centrifugation, filtration, treatment with a crystallized protein precipitating agent (salting out method), extraction, ultrasonic disruption, ultrafiltration, dialysis, various kinds of chromatography such as molecular sieve chromatography (gel filtration), absorption chromatography, ion exchange chromatography, affinity chromatography and the like, HPLC, or a combination of these methods may be used, and using a suitable method known in the art, targeted GMP may be recovered from a medium or microorganism.

The method for producing GMP of the present disclosure may comprise a purification step additionally. The purification may be performed, using an appropriate method known in the art. In one embodiment, when the method for producing GMP comprises both the recovery step and purification step, the recovery step and purification step may be performed continuously or non-continuously regardless of the order, or be performed simultaneously or as integrated into one step, but not limited thereto.

Other embodiment of the present disclosure is to provide a composition for producing GMP comprising a microorganism with enhanced activity of glutamine-hydrolyzing GMP synthase, or a medium in which the microorganism is cultured, or a combination thereof.

The microorganism with enhanced activity of glutamine-hydrolyzing GMP synthase and GMP are as described above.

Other embodiment provides a use for using in GMP production of the microorganism.

Other embodiment provides a use for using in preparation of a composition for producing GMP of the microorganism.

The composition of the present disclosure may further comprise any appropriate excipient commonly used for compositions for producing GMP, and such an excipient may be for example, a preservative, wetting agent, dispersant, suspending agent, buffer, stabilizer or tonicifying agent or the like, but not limited thereto.

### [ADVANTAGEOUS EFFECTS]

The present disclosure relates to a novel glutamine-hydrolyzing GMP synthase variant and a method for producing 5'-guanosine monophosphate using the same, and high-yield GMP production is possible by culturing a microorganism introduced with the glutamine-hydrolyzing GMP synthase variant of the present disclosure.

### [MODE FOR INVENTION]

Hereinafter, the present disclosure will be described in more detail by examples. However, the following examples are only preferable embodiments for illustrating the present disclosure, and thus, they are not intended to limit the scope of the present disclosure. On the other hand, technical matters not described in the present description can be sufficiently understood and easily implemented by those skilled in the technical field of the present disclosure or similar technical fields.

### Example 1: Identification of mutations for enhancing the activity of glutamine-hydrolyzing GMP synthase

The activity of glutamine-hydrolyzing GMP synthase (GuaA protein) encoded by guaA gene (SEQ ID NO: 2), a protein intrinsically present in microorganisms of the genus *Corynebacterium* was enhanced to identify protein mutations increasing the GMP conversion efficiency according to the following examples.

### Example 1-1: Construction of vector comprising guaA

In order to construct guaA library for identifying mutations that enhance the activity of GuaA predicted to be involved in GMP conversion efficiency, first, pCES208-Pn vector comprising a promoter of guaA and guaA upstream nucleotides 500bp (SEQ ID NO: 51) was constructed.

Specifically, chromosomal genes of the wild-type *Corynebacterium stationis* ATCC6872 strain were isolated using G-spin Total DNA extraction mini kit (Cat. No 17045) of Intron company according to the protocol provided in the kit, and guaA gene fragments were obtained by performing polymerase chain reaction using the primer pair of SEQ ID NO: 3 and SEQ ID NO: 4. It was performed under PCR amplification conditions of denaturation at 95°C for 5 minutes, and then repeating denaturation at 95°C for 30 seconds, annealing at 55°C for 30 seconds, and polymerization at 72°C for 40 seconds 25 times, and then polymerization at 72°C for 5 minutes.

The gene fragments obtained above were mixed with the pCES208 vector prepared by cutting with XbaI, BamHI restriction enzymes and were cloned using Gibson assembly (DG Gibson et al., NATURE METHODS, VOL.6 NO.5, MAY 2009, NEBuilder HiFi DNA Assembly Master Mix) method to obtain a recombinant plasmid, and this was named pCES208-Pn.

On the other hand, a vector to be used as a control group for guaA random mutation evaluation was constructed by the following method.

Specifically, gene fragments of SEQ ID NO: 1 for constructing the vector were obtained by PCR using chromosomal DNA of the *Corynebacterium stationis* ATCC6872 strain as a template. PCR products were obtained using the primers of SEQ ID NO: 5 and SEQ ID NO: 6, and as polymerase, SolgTM Pfu-X DNA polymerase was used. As PCR amplification conditions, denaturation at 95°C for 5 minutes, repeating denaturation at 95°C for 30 seconds, annealing at 55°C for 30 seconds, and polymerization at 72°C for 2 minutes 25 times, and polymerization at 72°C for 5 minutes were performed to obtain PCR products. The amplified products were mixed with the pCES208-Pn vector previously prepared by cutting it with XbaI, BamHI restriction enzymes and were cloned using Gibson assembly (DG Gibson et al., NATURE METHODS, VOL.6 NO.5, MAY 2009, NEBuilder HiFi DNA Assembly Master Mix) method to obtain a recombinant plasmid, and this was named pCES208-Pn-guaA(wt).

The sequences of the primers used in Example 1-1 were shown in Table 1 below.

**[Table 1]**

| Name | Sequence (5'->3') | SEQ ID NO |
|---|---|---|
| pCES208-Pn-F | GGTATCGATAAGCTTGATATCGAATTCCTGCAG CCCGGGGTGGCAGTAGCTGAAATCATT | SEQ ID NO: 3 |
| pCES208-Pn-R | GGCGAATTGGAGCTCCACCGCGGTGGCGGCCGC TCTAGAACTTGGATCCCACAGGTAGTTTAACAC ACCC | SEQ ID NO: 4 |
| pCES208-Pn-guaA(wt)-F | GTTTACCGCCGTAGGCCGCGGGTGtGTTAAACTA CCTGTATGACTCAACCTGCAACAACT | SEQ ID NO: 5 |
| pCES208-Pn-guaA(wt)-R | CTATAGGGCGAATTGGAGCTCCACCGCGGTGGC GGCCGCTTTACTCCCACTCGATGGTTC | SEQ ID NO: 6 |

### Example 1-2: Construction of guaA mutation library

A guaA mutation library vector was constructed based on the vector constructed in Example 1-1 by the following method. Gene fragments for constructing a random library vector were obtained by PCR using chromosomal DNA of the *Corynebacterium stationis* ATCC6872 strain as a template.

Specifically, a vector, in which a polynucleotide encoding guaA gene in which a mutation was introduced into the endogenous guaA gene site of *Corynebacterium stationis* could be substituted, was constructed, and as library, error-prone PCR kit (clontech Diversify^{®} PCR Random Mutagenesis Kit) was used, and a mutation was randomly induced according to the manufacturer's manual using the primers of SEQ ID NO: 5 and SEQ ID NO: 6 to obtain guaA mutant PCR products having different sequences from each other. The amplified products were mixed with the pCES208-Pn vector previously prepared by cutting it with XbaI, BamHI restriction enzymes and were cloned using Gibson assembly (DG Gibson et al., NATURE METHODS, VOL.6 NO.5, MAY 2009, NEBuilder HiFi DNA Assembly Master Mix) method to obtain a recombinant plasmid, and this was named pCES208-Pn-guaA random library.

### Example 1-3: Evaluation of constructed guaA random library and selection of strain

The pCES208-Pn-guaA random library constructed in Example 1-2 was transformed to a GMP converting strain, KCCM13320P (KR 10-2024-0133212 A) or *Corynebacterium stationis* ATCC6872 strain by electroporation (Appl. Microbiol.Biotechnol. (1999) 52:541-545), and then 10,000 strain colonies in which mutant genes were inserted were obtained by spreading on a seed medium containing 25 mg/L kanamycin and 2% agar, and each colony was named pCES208-Pn-guaA(mt)_1 to pCES208-Pn-guaA(mt)_10000.

The obtained 10,000 colonies were inoculated into a 96 Deep Well Plate, in which a 350 *µ*ℓ seed medium comprising kanamycin (25 mg/L) corresponding to a filling rate of 17% per each well was dispensed, using Qpix420 equipment, Colony Picker equipment of Molecular Devices company, and the *Corynebacterium stationis* ATCC6872 strain comprising a vector encoding wild-type guaA, pCES208-Pn-guaA(wt) was inoculated into 4 wells per plate in the same plate, and it was used as a screening control group for variant with high GMP conversion efficiency. The 96 Deep Well Plate in which the control strain and variant were inoculated respectively, was sealed using Gas permeable seal mark2 of Azenta company, and then cultured in Multitron equipment, a shaking incubator of Infors-HT company, under conditions of 30°C, 1,000 rpm for 48 hours. 1% xylene was added to the cultured strain and variant and additional culturing was performed for 1 hour, and then 150 *µ*ℓ of the titer evaluation solution comprising XMP was added and additional culture reaction was performed in Multitron equipment, a shaking incubator of Infors-HT company, under conditions of 30°C, 1,000 rpm for 12 hours. The 96 Deep Well Plate cultured for 12 hours was centrifuged in Centrifuge 5810R equipment, a centrifuge of Eppendorf company under conditions of 15°C, 4,000 rpm for 20 minutes, and then for NIR Spectrometry, 100 *µ*ℓ of the culture supernatant form which microbial cells were isolated was transferred to a 96 Well Black Polystyrene Microplate of Corning company using Biomek i5, a Liquid handler equipment of Beckman Coulter company. After that, individual analysis spectra of each well were obtained by applying to NIR Spectrometry, and through preliminary HPLC analysis, based on culture samples of which GMP concentrations were quantified, by applying to a regression analysis prediction model with a coefficient of determination of 0.96 for the established GMP 0 ~ 20 g/L concentration section, a selection logic of 15% or more improvement in GMP concentration compared to the control group was applied, and thereby, 6 strains among 10,000 variants were primarily selected. Culturing was performed for the selected 6 kinds by the same method, thereby finally selecting 2 kinds of strains with high GMP concentrations.

The compositions of the seed medium and titer evaluation solution used in Example 1-3 were as follows:

### <Seed medium>

Glucose 30 g/L, peptone 15 g/L, yeast extract 15 g/L, sodium chloride 2.5 g/L, urea 3 g/L, adenine 150 mg/L, guanine 150 mg/L, agar 20 g/L, pH 7.0 (based on 1 liter of distilled water)

### <Titer evaluation solution>

Trizma-base 24.20 g/L, ATP 3 0g/L, XMP · 2Na · 7H₂O 30 g/L, magnesium sulfate 15 g/L, ammonium sulfate 20 g/L

### Example 1-4: Confirmation of guaA mutation by gene sequencing

In order to confirm guaA gene mutant sequences of the two kinds of mutant strains selected in Example 1-3, PCR and sequencing were performed in the pCES208-Pn-guaA(mt)_2338, pCES208-Pn-guaA(mt)_3450 strains using the primer pair of SEQ ID NO: 7 and SEQ ID NO: 8, and they were compared with the guaA gene sequence of the wild-type *Corynebacterium stationis* ATCC6872 strain.

As a result of comparing with the guaA gene sequence of the wild-type strain, it was confirmed that the pCES208-Pn-guaA(mt)_2338 strain comprised a mutation in which the 123rd amino acid of the amino acid sequence encoded by guaA gene (SEQ ID NO: 1) was substituted from aspartic acid(D) to lysine (K), and the pCES208-Pn-guaA(mt)_3450 strain comprised a mutation in which the 441st amino acid of the amino acid sequence encoded by guaA gene was substituted from aspartic acid (D) to valine (V), as described above.

The sequences of the primers used in Example 1-4 were shown in Table 2 below.

**[Table 2]**

| Name | Sequence (5'->3') | SEQ ID NO |
|---|---|---|
| pCES208-Pn-guaA seq-F | TTCGAGCTCGGTACCCGTCAGCAGTGGAACGAA GGCGAC | SEQ ID NO: 7 |
| pCES208-Pn-guaA seq-R | CTCTAGAGGATccccAGATATCGGTCAGGTGGTC ATCG | SEQ ID NO: 8 |

In the following example, whether the guaA mutations affected the GMP conversion efficiency was to be confirmed.

### Example 2: Construction of strains introduced with guaA variants and evaluation of GMP production capacity (conversion efficiency)

### Example 2-1: Construction of recombinant vector for guaA variant introduction

In order to confirm the influence of the D123K mutation and D441V mutation identified in Example 1-4 on GMP production capacity (conversion efficiency), a vector introducing the mutation into an endogenous guaA gene of a *Corynebacterium stationis* strain was constructed.

At first, in order to confirm the effect of the mutant guaA (D123K) in which aspartic acid at the 123rd position of the amino acid sequence of the GuaA protein was substituted with lysine on GMP conversion efficiency, a vector for constructing a strain expressing thereof was constructed using the plasmid pDC244(SEQ ID NO: 112) for insertion and replacement of genes in *Corynebacterium* chromosome as follows.

PCR was performed using the pCES208-Pn-guaA(mt)_2338 vector as a template using the primer pair of SEQ ID NO: 9 and SEQ ID NO: 10. As polymerase, SolgTM Pfu-X DNA polymerase was used, and as PCR amplification conditions, denaturation at 95°C for 5 minutes, and then repeating denaturation at 95°C for 30 seconds, annealing at 55°C for 30 seconds, and polymerization at 72°C for 2 minutes 25 times, and then polymerization at 72°C for 5 minutes, to obtain PCR products. The obtained PCR products were mixed with the pDC24 vector prepared by previously cutting it with XbaI restriction enzyme and were cloned using Gibson assembly (DG Gibson et al., NATURE METHODS, VOL.6 NO.5, MAY 2009, NEBuilder HiFi DNA Assembly Master Mix) method, to obtain a recombinant plasmid, and this was named pDC24-guaA(D123K).

By the same method, in order to confirm the effect of the mutant guaA (D441V) in which aspartic acid at the 441st position of the amino acid sequence of the GuaA protein was substituted with valine on GMP conversion efficiency, PCR products were obtained using the pCES208-Pn-guaA(mt)_3450 vector as a template and using the primer pair of SEQ ID NO: 9 and SEQ ID NO: 10, and then were cloned in the pDC24 vector cut with XbaI to construct a recombinant plasmid, and this was named pDC24-guaA(D441V).

The sequences of the primers used in Example 2-1 were shown in Table 3 below.

**[Table 3]**

| Name | Sequence (5'->3') | SEQ ID NO |
|---|---|---|
| pDC24-guaA(mt)-F | acgacggccagtgaattcgagctcggtacccggggatcctTGGCAGT AGCTGAAATCATT | SEQ ID NO: 9 |
| pDC24-guaA(mt)-R | gaccatgattacgccaagcttgcatgcctgcaggtcgactTTACTCCCA CTCGATGGTTC | SEQ ID NO: 10 |

### Example 2-2: Construction of strains expressing guaA variants

In order to confirm the influence of the D123K mutation and D441V mutation identified in Example 1-4 on GMP production capacity (conversion efficiency), strains introduced with endogenous guaA genes of a *Corynebacterium stationis* strain were constructed.

The pDC24-guaA(D123K), pDC24-guaA(D441V) vectors constructed in Example 2-1 were transformed respectively, into GMP converting strains, *Corynebacterium stationis* KCCM13320P, respectively, by electroporation, and then strains in which the vector was inserted into chromosome by recombination of homologous sequences in a selection medium containing 25 mg/L kanamycin were selected as a primary candidate group. The selected primary strains were passed through secondary cross-over again, and strains introduced with a mutation of a target gene were selected. Whether a genetic mutation of the finally transformed strains was introduced was confirmed by sequencing using SEQ ID NO: 11, SEQ ID NO: 12, after performing PCR using the primer pair of SEQ ID NO: 11 and SEQ ID NO: 12. The obtained strains were named KCCM13320P::guaA(D123K) or ATCC6872::guaA(D123K), KCCM13320P::guaA(D441V) or ATCC6872::guaA(D441V), respectively.

The sequences of the primers used in Example 2-2 were shown in Table 4 below.

**[Table 4]**

| Name | Sequence (5'->3') | SEQ ID NO |
|---|---|---|
| pDC24-guaA seq-F | TGTGATTGCCGGTGCCAGCA | SEQ ID NO: 11 |
| pDC24-guaA seq-R | CCTACTAAAGGCGAAGCCCC | SEQ ID NO: 12 |

### Example 2-3: Evaluation of GMP production capacity of strains expressing guaA variants

In order to confirm GMP conversion efficiency of 2 kinds of strains of KCCM13320P::guaA(D123K), KCCM13320P::guaA(D441V) produced in Example 2-2, they were cultured by the following method.

Specifically, a parent strain, *Corynebacterium stationis* KCCM13320P and the 2 kinds of mutant strains produced in Example 2-2 were inoculated in a 250 ml corner-baffled flask containing the seed medium of 25 ml described in Example 1-3, respectively, and then they were cultured with shaking at 200rpm at 30°C for 20 hours. 800 *µ*ℓ of the titer evaluation solution of described in Example 1-3 was added to 200 *µ*ℓ of the cultured solution, and they were reacted at 42°C for 30 minutes to convert XMP into GMP. After completing culturing, the produced amount of GMP was measured using liquid high-performance chromatography, and the GMP concentration in the cultured solution for each strain experimented was shown in Table 5 below.

**[Table 5]**

| Comparison of GMP conversion efficiency of *Corynebacterium stationis* KCCM13320P, KCCM13320P::guaA(D123K), KCCM13320P::guaA(D441V) | | | |
|---|---|---|---|
| Strain number | XMP concentration (g/l) | GMP concentration (g/l) | Increase/decrease rate of GMP production compared to parent strain (%) |
| KCCM13320P (parent strain) | 8.30 | 2.06 | - |
| KCCM13320P:: guaA(D123K) | 7.10 | 3.28 | 159% |
| KCCM13320P:: guaA(D441V) | 6.53 | 3.83 | 186% |

As a result, as shown in Table 5, it was confirmed that the parent strain, *Corynebacterium stationis* (*Corynebacterium ammoniagenes*) KCCM13320P produced (converted) GMP at a concentration of 2.06g/l, but the mutant strains in which the mutation was introduced into GuaA protein, KCCM13320P::guaA(D123K), KCCM13320P::guaA(D441V) converted GMP at a concentration of 3.28 g/l, 3.83 g/l, respectively.

Through the results, it was confirmed that the strains in which a mutation in which the 123rd amino acid of the amino acid sequence encoding guaA gene was substituted with lysine or a mutation in which the 441st amino acid was substituted with valine showed an increase in the GMP produced amount compared to the strain in which the mutation was not introduced.

### Example 2-4: Evaluation of GDP production capacity of strains expressing guaA variants

In order to confirm an effect of increasing a GMP synthesis pathway factor, GDP production capacity, in addition to GMP conversion efficiency of the 2 kinds of mutant strains of KCCM13320P::guaA(D123K), KCCM13320P::guaA(D441V) produced in Example 2-2, they were cultured by the same method as Example 2-3, and 800 *µ*ℓ of the titer evaluation solution described in Example 1-3 was added to 200 *µ*ℓ of the cultured solution and they were reacted at 42°C for 30 minutes to convert XMP into GMP. After completing culturing, the produced amounts of GMP and GDP were measured using liquid high-performance chromatography, and the concentrations were shown in Table 6 below.

**[Table 6]**

| Strain number | XMP concentration (g/l) | GMP concentration (g/l) | GDP concentration (g/l) |
|---|---|---|---|
| KCCM13320P (parent strain) | 6.30 | 2.01 | 0.31 |
| KCCM13320P:: guaA(D123K) | 5.10 | 3.14 | 0.48 |
| KCCM13320P:: guaA(D441V) | 4.53 | 3.69 | 0.52 |

As a result, as shown in Table 6 above, it was confirmed that the parent strain, *Corynebacterium stationis* KCCM13320P produced GMP at a concentration of 2.01 g/l, and produced GDP at a concentration of 0.31g/l, and the mutant strains in which the mutation was introduced into GuaA protein, KCCM13320P::guaA(D123K), KCCM13320P::guaA(D441V) converted GMP at a concentration of 3.14 g/l, 3.69 g/l, respectively, and produced GDP at a concentration of 0.48 g/l, 0.52 g/l, respectively.

Through the results, it was confirmed that the strains in which a mutation in which the 123rd amino acid of the amino acid sequence encoding guaA gene was substituted with lysine or a mutation in which the 441st amino acid was substituted with valine showed an increase in the GMP production capacity (conversion efficiency) and GDP production capacity compared to the strain in which the mutation was not introduced.

### Example 3: Substitution of amino acid of GuaA(D123K) mutation with another amino acid

### Example 3-1: Construction of vectors for inserting substitution of GuaA(D123) mutation amino acid

Through Example 2 above, it was confirmed that the GMP conversion efficiency could be improved by the GuaA(D123) position mutation. Accordingly, to confirm the positional importance of GuaA(D123), vectors substituting the 123rd amino acid with another amino acid were produced, and whether they affected the GMP conversion efficiency was confirmed.

Site-directed mutagenesis was performed using the pDC24-guaA(D123K) vector constructed in Example 2-1 as a template. Specifically, using the primer pairs of SEQ ID NO: 9 and SEQ ID NO: 13 and SEQ ID NO: 14 and SEQ ID NO: 10 for introduction of GuaA(D123E) mutation, the primer pairs of SEQ ID NO: 9 and SEQ ID NO: 15 and SEQ ID NO: 16 and SEQ ID NO: 10 for introduction of GuaA(D123H) mutation, the primer pairs of SEQ ID NO: 9 and SEQ ID NO: 17 and SEQ ID NO: 18 and SEQ ID NO: 10 for introduction of GuaA(D123M) mutation, the primer pairs of SEQ ID NO: 9 and SEQ ID NO: 19 and SEQ ID NO: 20 and SEQ ID NO: 10 for introduction of GuaA(D123Q) mutation, the primer pairs of SEQ ID NO: 9 and SEQ ID NO: 21 and SEQ ID NO: 22 and SEQ ID NO: 10 for introduction of GuaA(D123R) mutation, the primer pairs of SEQ ID NO: 9 and SEQ ID NO: 23 and SEQ ID NO: 24 and SEQ ID NO: 10 for introduction of GuaA(D123S) mutation, and the primer pairs of SEQ ID NO: 9 and SEQ ID NO: 25 and SEQ ID NO: 26 and SEQ ID NO: 10 for introduction of GuaA(D123Y) mutation, site-directed PCR was performed, respectively, and then, as polymerase, SolgTM Pfu-X DNA polymerase was used, and as PCR amplification conditions, denaturation at 95°C for 5 minutes, and then repeating denaturation at 95°C for 30 seconds, annealing at 55°C for 30 seconds, and polymerization at 72°C for 1 minute and 30 seconds 25 times, and then polymerization at 72°C for 5 minutes were performed, to obtain each of PCR products. Each of the amplified products was treated with DpnI restriction enzyme to remove the pDC24-guaA(D123K) used as a template, and then it was transformed into DH5a to obtain plasmids in which the GuaA 123rd amino acid was modified into the targeted amino acid.

The information of the obtained plasmids was shown in Table 7 below:

**[Table 7]**

| List of vectors for inserting substitution of GuaA(D123) mutation amino acid | |
|---|---|
| Number | Plasmid name |
| 1 | pDC24-guaA(D123E) |
| 2 | pDC24-guaA(D123H) |
| 3 | pDC24-guaA(D123M) |
| 4 | pDC24-guaA(D123Q) |
| 5 | pDC24-guaA(D123R) |
| 6 | pDC24-guaA(D123S) |
| 7 | pDC24-guaA(D123Y) |

### Example 3-2: Construction of strains having mutation of GuaA 123rd amino acid variant substituted with another amino acid and confirmation of GMP conversion efficiency

7 kinds of the vectors for introducing mutation produced in Example 3-1 were transformed into *Corynebacterium stationis* KCCM13320P strain, respectively, by electroporation, and the strains in which the vectors were inserted on chromosome by recombination of homologous sequences were selected in a medium containing 25 mg/L kanamycin. The primary strains selected were passed through secondary cross-over again, and strains in which mutation was introduced into a target gene were selected. The introduction of genetic mutation in the final transformed strains was confirmed through PCR using the primer pair of SEQ ID NO: 11 and SEQ ID NO: 12 and sequencing, and the strain names according to the inserted mutation were shown in Table 8 below:

**[Table 8]**

| List of strains having mutation of GuaA 123rd amino acid variant substituted with another amino acid | |
|---|---|
| No | Strain name |
| 1 | KCCM13320P::guaA(D123E) |
| 2 | KCCM13320P::guaA(D123H) |
| 3 | KCCM13320P::guaA(D123M) |
| 4 | KCCM13320P::guaA(D123Q) |
| 5 | KCCM13320P::guaA(D123R) |
| 6 | KCCM13320P::guaA(D123S) |
| 7 | KCCM13320P::guaA(D123Y) |

The 7 kinds of strains produced above and KCCM13320P and KCCM13320P::guaA(D123K) strains were cultured by the same method as Example 2-3 to evaluate the conversion efficiency of GMP. The results of evaluation were shown in Table 9 below:

**[Table 9]**

| Results of evaluation of GMP conversion efficiency of strains having mutation of GuaA 123rd amino acid variant substituted with another amino acid | | | |
|---|---|---|---|
| Strain number | XMP concentration (g/l) | GMP concentration (g/l) | Increase/decrease rate of GMP production compared to parent strain (%) |
| KCCM13320P(parent strain) | 8.40 | 2.63 | - |
| KCCM13320P::guaA(D123K) | 7.03 | 3.98 | 151% |
| KCCM13320P::guaA(D123E) | 8.23 | 2.72 | 103% |
| KCCM13320P::guaA(D123H) | 8.08 | 2.67 | 101% |
| KCCM13320P::guaA(D123M) | 7.96 | 2.65 | 101% |
| KCCM13320P::guaA(D123Q) | 8.15 | 2.77 | 105% |
| KCCM13320P::guaA(D123R) | 8.17 | 2.79 | 106% |
| KCCM13320P::guaA(D123S) | 8.27 | 2.66 | 101% |
| KCCM13320P::guaA(D123Y) | 8.32 | 2.72 | 103% |

As a result, as shown in Table 9, it could be confirmed that the strains comprising the guaA mutation gene in which the 123rd amino acid of the amino acid sequence encoded by the guaA gene was substituted with another amino acid, had an increased produced amount of GMP compared to KCCM13320P not comprising the mutation (parent strain), in the GMP producing strains. In other words, it was confirmed that the 123rd amino acid of the amino acid sequence encoded by the guaA gene was an important mutation position in GMP production (conversion). More specifically, it was confirmed that the GMP production capacity (conversion efficiency) of the microorganisms comprising a mutation in which the 123rd amino acid of the amino acid sequence encoded by the guaA gene was substituted with lysine, glutamic acid, histidine, methionine, glutamine, arginine, serine or tyrosine was significantly increased.

### Example 4: Substitution of amino acid of GuaA(D441V) mutation into another amino acid

### Example 4-1: Construction of vectors for inserting substitution of GuaA(D441) mutation amino acid

Through Example 2 above, it was confirmed that the GMP conversion efficiency could be improved by the GuaA(D441) position mutation. Accordingly, to confirm the positional importance of GuaA(D441), vectors substituting the 123rd amino acid with another amino acid were produced, and whether they affected the GMP conversion efficiency was confirmed.

Site-directed mutagenesis was performed using the pDC24-guaA(D441V) vector constructed in Example 2-1 as a template. Specifically, using the primer pairs of SEQ ID NO: 9 and SEQ ID NO: 27 and SEQ ID NO: 28 and SEQ ID NO: 10 for introduction of GuaA(D441A) mutation, the primer pairs of SEQ ID NO: 9 and SEQ ID NO: 29 and SEQ ID NO: 30 and SEQ ID NO: 10 for introduction of GuaA(D441C) mutation, the primer pairs of SEQ ID NO: 9 and SEQ ID NO: 31 and SEQ ID NO: 32 and SEQ ID NO: 10 for introduction of GuaA(D441E) mutation, the primer pairs of SEQ ID NO: 9 and SEQ ID NO: 33 and SEQ ID NO: 34 and SEQ ID NO: 10 for introduction of GuaA(D441F) mutation, the primer pairs of SEQ ID NO: 9 and SEQ ID NO: 35 and SEQ ID NO: 36 and SEQ ID NO: 10 for introduction of GuaA(D441H) mutation, the primer pairs of SEQ ID NO: 9 and SEQ ID NO: 37 and SEQ ID NO: 38 and SEQ ID NO: 10 for introduction of GuaA(D441I) mutation, and the primer pairs of SEQ ID NO: 9 and SEQ ID NO: 39 and SEQ ID NO: 40 and SEQ ID NO: 10 for introduction of GuaA(D441K) mutation, the primer pairs of SEQ ID NO: 9 and SEQ ID NO: 41 and SEQ ID NO: 42 and SEQ ID NO: 10 for introduction of GuaA(D441L) mutation, the primer pairs of SEQ ID NO: 9 and SEQ ID NO: 43 and SEQ ID NO: 44 and SEQ ID NO: 10 for introduction of GuaA(D441N) mutation, the primer pairs of SEQ ID NO: 9 and SEQ ID NO: 45 and SEQ ID NO: 46 and SEQ ID NO: 10 for introduction of GuaA(D441Q) mutation, the primer pairs of SEQ ID NO: 9 and SEQ ID NO: 47 and SEQ ID NO: 48 and SEQ ID NO: 10 for introduction of GuaA(D441T) mutation, and the primer pairs of SEQ ID NO: 9 and SEQ ID NO: 49 and SEQ ID NO: 50 and SEQ ID NO: 10 for introduction of GuaA(D441W) mutation, site-directed PCR was performed, respectively, and then, as polymerase, SolgTM Pfu-X DNA polymerase was used, and as PCR amplification conditions, denaturation at 95°C for 5 minutes, and then repeating denaturation at 95°C for 30 seconds, annealing at 55°C for 30 seconds, and polymerization at 72°C for 1 minute and 30 seconds 25 times, and then polymerization at 72°C for 5 minutes were performed, to obtain each of PCR products. Each of the amplified products was treated with DpnI restriction enzyme to remove the pDC24-guaA(D123K) used as a template, and then it was transformed into DH5a to obtain plasmids in which the GuaA 441st amino acid was modified into the targeted amino acid.

The information of the obtained plasmids was shown in Table 10 below:

**[Table 10]**

| List of vectors for inserting substitution of GuaA(D441) mutation amino acid | |
|---|---|
| No | Plasmid name |
| 1 | pDC24-guaA(D441A) |
| 2 | pDC24-guaA(D441C) |
| 3 | pDC24-guaA(D441E) |
| 4 | pDC24-guaA(D441F) |
| 6 | pDC24-guaA(D441H) |
| 7 | pDC24-guaA(D441I) |
| 8 | pDC24-guaA(D441K) |
| 9 | pDC24-guaA(D441L) |
| 11 | pDC24-guaA(D441N) |
| 13 | pDC24-guaA(D441Q) |
| 16 | pDC24-guaA(D441T) |
| 17 | pDC24-guaA(D441W) |

### Example 4-2: Construction of strains having mutation of GuaA 441st amino acid variant substituted with another amino acid and confirmation of GMP conversion efficiency

18 kinds of the vectors for introducing mutation produced in Example 4-1 were transformed into *Corynebacterium stationis* KCCM13320P strain, respectively, by electroporation, and the strains in which the vectors were inserted on chromosome by recombination of homologous sequences were selected in a medium containing 25 mg/L kanamycin. The primary strains selected were passed through secondary cross-over again, and strains in which mutation was introduced into a target gene were selected. The introduction of genetic mutation in the final transformed strains was confirmed through PCR using the primer pair of SEQ ID NO: 11 and SEQ ID NO: 12 and sequencing, and the strain names according to the inserted mutation were shown in Table 11 below:

**[Table 11]**

| List of strains having mutation of GuaA 441st amino acid variant substituted with another amino acid | |
|---|---|
| No | Strain name |
| 1 | KCCM13320P::guaA(D441A) |
| 2 | KCCM13320P::guaA(D441C) |
| 3 | KCCM13320P::guaA(D441E) |
| 4 | KCCM13320P::guaA(D441F) |
| 6 | KCCM13320P::guaA(D441H) |
| 7 | KCCM13320P::guaA(D441I) |
| 8 | KCCM13320P::guaA(D441K) |
| 9 | KCCM13320P::guaA(D441L) |
| 11 | KCCM13320P::guaA(D441N) |
| 13 | KCCM13320P::guaA(D441Q) |
| 16 | KCCM13320P::guaA(D441T) |
| 17 | KCCM13320P::guaA(D441W) |

The 18 kinds of strains produced above and KCCM13320P and KCCM13320P::guaA(D441V) strains were cultured by the same method as Example 2-3 to evaluate the conversion efficiency of GMP. The results of evaluation were shown in Table 12 below:

**[Table 12]**

| Results of evaluation of GMP conversion efficiency of strains having mutation of GuaA 441st amino acid variant substituted with another amino acid | | | |
|---|---|---|---|
| Strain number | XMP concentration (g/l) | GMP concentration (g/l) | Increase/decrease rate of GMP production compared to parent strain (%) |
| KCCM13320P(parent strain) | 8.29 | 2.88 | - |
| KCCM13320P::guaA(D441V) | 4.92 | 7.05 | 245% |
| KCCM13320P::guaA(D441A) | 8.08 | 3.06 | 106% |
| KCCM13320P::guaA(D441C) | 5.98 | 5.46 | 190% |
| KCCM13320P::guaA(D441E) | 6.61 | 4.96 | 172% |
| KCCM13320P::guaA(D441F) | 6.35 | 5.06 | 176% |
| KCCM13320P::guaA(D441H) | 7.81 | 4.01 | 139% |
| KCCM13320P::guaA(D441I) | 5.10 | 6.72 | 234% |
| KCCM13320P::guaA(D441K) | 7.44 | 4.50 | 157% |
| KCCM13320P::guaA(D441L) | 5.79 | 5.59 | 194% |
| KCCM13320P::guaA(D441N) | 5.58 | 6.07 | 211% |
| KCCM13320P::guaA(D441Q) | 5.99 | 5.31 | 185% |
| KCCM13320P::guaA(D441T) | 8.05 | 3.67 | 127% |
| KCCM13320P::guaA(D441W) | 8.17 | 3.26 | 113% |

As a result, as shown in Table 12, it could be confirmed that the strains comprising the guaA mutation gene in which the 441st amino acid of the amino acid sequence encoded by the guaA gene was substituted with another amino acid, had an increased produced amount of GMP compared to KCCM13320P not comprising the mutation (parent strain), in the GMP producing strains. In other words, it was confirmed that the 441st amino acid of the amino acid sequence encoded by the guaA gene was an important mutation position in GMP production (conversion). More specifically, it was confirmed that the GMP production capacity (conversion efficiency) of the microorganisms comprising a mutation in which the 441st amino acid of the amino acid sequence encoded by the guaA gene was substituted with valine, alanine, cysteine, glutamic acid, phenylalanine, histidine, isoleucine, lysine, leucine, asparagine, glutamine, threonine, or tryptophan was significantly increased.

### Example 5: Confirmation of effects of GuaA protein D123K + D441V combination variant

### Example 5-1: Production of strain expressing GuaA protein D123K + D441V combination variant

Into the KCCM13320P::guaA(D123K) strain produced in Example 2-2, the pDC24-guaA(D441V) vector constructed in Example 2-1 was transformed by electroporation, and strains in which vectors were inserted on chromosome by recombination of homologous sequences were selected in a medium containing 25 mg/L kanamycin. The selected primary strains were passed through secondary cross-over again, and a strain in which mutation of a target gene was introduced was selected. The introduction of genetic mutation of the final transformed strain was confirmed by performing PCR using the primer pair of SEQ ID NO: 11 and SEQ ID NO: 12, and then sequencing using SEQ ID NO: 11, SEQ ID NO: 12, and the obtained strain was named KCCM13320P::guaA(D123K, D441V).

### Example 5-2: Evaluation of GMP production capacity of strain expressing GuaA protein D123K + D441V combination variant

In order to confirm the GMP conversion efficiency of the 2 kinds of mutant strains of KCCM13320P::guaA(D123K) and KCCM13320P::guaA(D441V) produced in Example 2-2 and the KCCM13320P::guaA(D123K, D441V) mutant strain produced in Example 5-1, each strain was cultured by the method of Example 2-3 to analyze the conversion efficiency of GMP. The analysis results were shown in Table 13 below:

**[Table 13]**

| Results of evaluation of GMP conversion efficiency of *Corynebacterium stationis* KCCM13320P, KCCM13320P::guaA(D123K), KCCM13320P::guaA(D441V), KCCM13320P::guaA(D123K, D441V) | | | |
|---|---|---|---|
| Strain number | XMP concentration (g/l) | GMP concentration (g/l) | Increase/decrease rate of GMP production compared to parent strain (%) |
| KCCM13320P(parent strain) | 7.94 | 2.16 | 100% |
| KCCM13320P::guaA(D123K) | 6.56 | 3.31 | 153% |
| KCCM13320P::guaA(D441V) | 5.77 | 3.89 | 180% |
| KCCM13320P::guaA(D123K, D441V) | 5.01 | 4.72 | 218% |

As a result, as shown in Table 13, it was confirmed that the parent strain, of *Corynebacterium stationis* KCCM13320P produced (converted) GMP at a guaA (concentration of 2.16 g/l, but the mutant strains in which mutation was introduced into GuaA protein, KCCM13320P::guaA(D123K), KCCM13320P::guaA(D441V) converted GMP at a concentration of 3.31 g/l, 3.89 g/l, respectively, and the KCCM13320P::guaA(D123K, D441V) strain introduced with the D123K + D441V combination mutation of GuaA protein converted GMP at a concentration of 4.72 g/l.

### Example 6: Confirmation of variant effects in guaA of another species of the genus Corynebacterium

In order to confirm whether the D123K mutation and D441 mutation identified in Example 1-4 is a mutation that improves GMP production capacity (conversion efficiency) also in other microorganisms of the genus *Corynebacterium*, first, the effect of the mutations on GuaA protein (SEQ ID NO: 96) of *Corynebacterium casei* (C. ca) with sequence identity of about 95% to the GuaA protein of *Corynebacterium stationis* was to be confirmed.

### Example 6-1: Construction of vectors for expressing guaA variants

In order to confirm the effect of guaA(D123K) in which aspartic acid at the 123rd position of the GuaA protein amino acid sequence was substituted with lysine and guaA(E441V) on GMP conversion efficiency, vectors for producing strains expressing thereof were constructed using the plasmid pDC24 for insertion and replacement of the gene (SEQ ID NO: 97) in *Corynebacterium* chromosome (Korena Publication No. 10-20202-0136813) as follows.

The chromosomal genes of the wild-type *Corynebacterium casei* LMG S-19264 strain were isolated using G-spin Total DNA extraction mini kit (Cat. No 17045) of Intron company according to the protocol provided in the kit, and using them as a template, and using the primer pairs of SEQ ID NO: 98 and SEQ ID NO: 100 and SEQ ID NO: 101 and SEQ ID NO: 99 for introduction of GuaA(D123K) mutation, and the primer pairs of SEQ ID NO: 98 and SEQ ID NO: 102 and SEQ ID NO: 103 and SEQ ID NO: 99 for introduction of GuaA(E441V), site-directed mutagenesis was performed. Then, as polymerase, SolgTM Pfu-X DNA polymerase was used and as PCR amplification conditions, denaturation at 95°C for 5 minutes, repeating denaturation at 95°C for 30 seconds, annealing at 55°C for 30 seconds, and polymerization at 72°C for 1 minute and 30 seconds 25 times, and then polymerization at 72°C for 5 minutes were performed to obtain each of PCR products. The gene fragments obtained above were mixed with the pDC24 vector prepared by cutting it with XbaI restriction enzyme and were cloned using Gibson assembly (DG Gibson et al., NATURE METHODS, VOL.6 NO.5, MAY 2009, NEBuilder HiFi DNA Assembly Master Mix) method to obtain recombinant plasmids, and each was named pDC24-guaA(C.ca, D123K), pDC24-guaA(C.ca, E441V).

The information of the obtained plasmids was shown in Table 14 below:

**[Table 14]**

| List of vectors for inserting substitution of GuaA(D123) mutation amino acids | |
|---|---|
| No | Plasmid name |
| 1 | pDC24-guaA(C.ca, D123K) |
| 2 | pDC24-guaA(C.ca, E441V) |

The sequences of the primers used in Example 6-1 were shown in Table 15 below.

**[Table 15]**

| Name | Sequence (5'->3') | SEQ ID NO |
|---|---|---|
| pDC24-guaA(mt)-F | acgacggccagtgaattcgagctcggtacccggggatcct GAAGAGATCAAAGAACGCGC | SEQ ID NO: 98 |
| pDC24-guaA(mt)-R | gaccatgattacgccaagcttgcatgcctgcaggtcgact CACCCAGAGGGTCTATAACC | SEQ ID NO: 99 |
| pDC24-guaA(C.ca, D123K) | CGTGCGACATCCACACCTTGTGGTT GGCCTCCAAGCCtTtGTGCAGCACGC CACCGGTGT | SEQ ID NO: 100 |
| pDC24-guaA(C.ca, D123K) | aAaGGCTTGGAGGCCAACCACAA | SEQ ID NO: 101 |
| pDC24-guaA(C.ca, E441V) | CGGACATCGGCGAGAAGAACGACT GGGCACTGCCAGATcaCGTTATCCAG GCCGGCGTTG | SEQ ID NO: 102 |
| pDC24-guaA(C.ca, E441V) | tgATCTGGCAGTGCCCAGTCGT | SEQ ID NO: 103 |

### Example 6-2: Construction of strains expressing guaA variants of Corynebacterium casei

In order to confirm the influence of the lysine mutation at the 123 position and the valine mutation at the 441st position of guaA amino acid identified in Example 1-4 on GMP production capacity (conversion efficiency), strains in which mutation was introduced into endogenous guaA genes of *Corynebacterium casei* strains were constructed.

The pDC24-guaA(C.ca, D123K), pDC24-guaA(C.ca, E441V) vectors constructed in Example 6-1 were transformed into the *Corynebacterium casei* LMG S-19264 strain, respectively, by electroporation, and then strains in which the vectors were inserted on chromosome by recombination of homologous sequences in a selection medium containing 25 mg/L kanamycin were selected as a primary candidate group. The selected primary strains were passed through secondary cross-over again, and strains in which mutation of a target gene was introduced were selected. The introduction of genetic mutation of the final transformed strains was confirmed by performing PCR using the primer pair of SEQ ID NO: 104 and SEQ ID NO: 105, and then sequencing using SEQ ID NO: 104, SEQ ID NO: 105. The obtained strains were named LMG S-19264::guaA(D123K) or LMG S-19264::guaA(E441V), respectively.

In addition, into the LMG S-19264::guaA(D123K) strain, the pDC24-guaA(C.ca, E441V) vector constructed in Example 6-1 was transformed by electroporation, and the strains in which the vectors were inserted on chromosome by recombination of homologous sequences were selected in a medium containing 25 mg/L kanamycin. The selected primary strains were passed through secondary cross-over again, and a strain in which mutation of a target gene was introduced was selected. The introduction of genetic mutation of the final transformed strain was confirmed by performing PCR using the primer pair of SEQ ID NO: 104 and SEQ ID NO: 105 and then sequencing using SEQ ID NO: 104, SEQ ID NO: 105, and the obtained strain was named LMG S-19264::guaA(C.ca, D123K, E441V).

The sequences of the primers used in Example 6-2 were shown in Table 16 below.

**[Table 16]**

| Name | Sequence (5'->3') | SEQ ID NO |
|---|---|---|
| pDC24-guaA seq-F | GTGACTCAACCTGCAACAAC | SEQ ID NO: 104 |
| pDC24-guaA seq-R | TTACTCCCACTCGATGGTTC | SEQ ID NO: 105 |

### Example 6-3: Evaluation of GMP production capacity of strains expressing guaA variants of Corynebacterium casei

In order to confirm GMP conversion efficiency of the 3 kinds of strains, LMG S-19264::guaA(D123K), LMG S-19264::guaA(E441V), LMG S-19264::guaA(C.ca, D123K, D441V) produced in Example 2-2, they were cultured by the following method.

Specifically, the parent strain, *Corynebacterium casei* LMG S-19264 and the 3 kinds of the mutant strains produced in Example 6-2 were inoculated into a 250 ml corner-baffled flask containing the seed medium of 25 ml described in Example 1-3, respectively, and then they were cultured with shaking at 200 rpm at 30°C for 20 hours. 800 *µ*ℓ of the titer evaluation solution described in Example 1-3 was added to 200 *µ*ℓ of the cultured solution and they were reacted at 42°C for 30 minutes to convert XMP into GMP. After completing culturing, the produced amount of GMP was measured using liquid high-performance chromatography, and the GMP concentration in the cultured solution for each strain experimented was shown in Table 17 below.

**[Table 17]**

| Comparison of GMP conversion efficiency of *Corynebacterium casei* LMG S-19264, LMG S-19264::guaA(D123K), LMG S-19264::guaA(E441V), LMG S-19264::guaA(C.ca, D123K, E441V) | | | |
|---|---|---|---|
| Strain number | XMP concentration (g/l) | GMP concentration (g/l) | Increase/decrease rate of GMP production compared to parent strain (%) |
| LMG S-19264 (parent strain) | 8.59 | 0.94 | - |
| LMG S-19264:: guaA(D123K) | 8.33 | 1.18 | 126% |
| LMG S-19264:: guaA(E441V) | 8.23 | 1.33 | 141% |
| LMG S-19264:: guaA(D123K, E441V) | 8.01 | 1.59 | 169% |

As a result, as shown in Table 17, it was confirmed that the parent strain, *Corynebacterium casei* LMG S-19264 produced (converted) GMP at a concentration of 0.94g/l, but the mutant strains introduced with mutation into GuaA protein, LMG S-19264::guaA(D123K), LMG S-19264::guaA(E441V), LMG S-19264::guaA(C.ca, D123K, E441V) converted GMP at a concentration of 1.18 g/l, 1.33 g/l, 1.59g/l, respectively.

Through the results, it was confirmed that the strains introduced with the mutation in which the 123rd amino acid of the amino acid sequence encoded by the guaA gene was substituted with lysine or the mutation in which the 441st amino acid was substituted with valine had an increased produced amount of GMP compared to the strain not introduced with the mutation.

From the above description, those skilled in the art to which the present disclosure pertains will be able to understand that the present disclosurese may be implemented in other specific forms without changing the technical spirit or essential features thereof. In this regard, the examples described above should be understood as illustrative and non-limiting in all respects. The scope of the present disclosure should be construed that all changed or modified forms derived from the meaning and scope of the claims to be described later and equivalent concepts thereof rather than the above detailed description are included in the scope of the present disclosure.

### [ACCESSION NUMBER]

Name of Depository Authority: Korean Culture Center of Microorganisms (KCCM)
Accession number: KCCM13320P
Date of deposit: 20230110

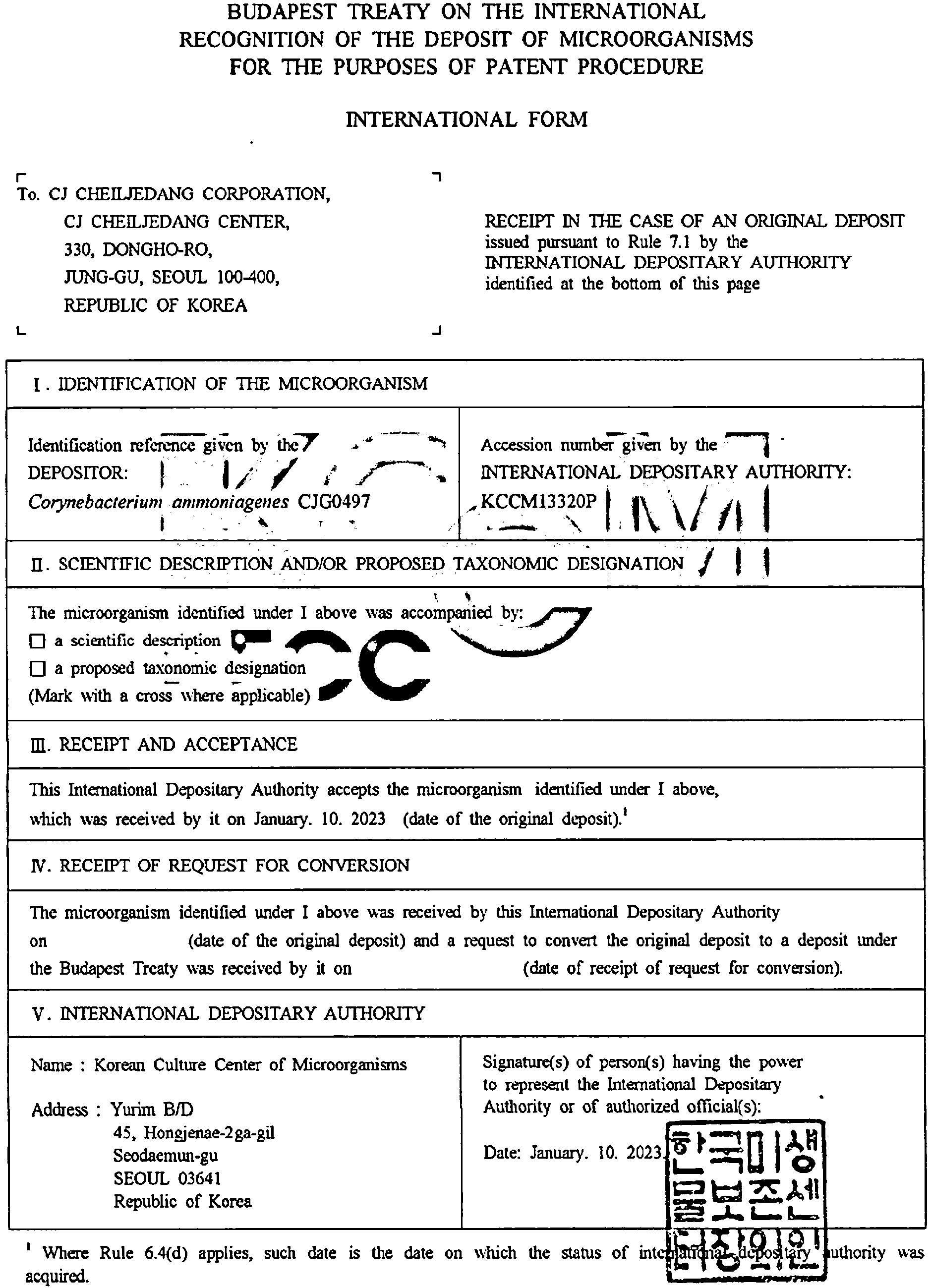
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium ammoniagenes CJG0497 | Accession number given by the
INTERNATIONAL DEPOSITARY AUTHORITY:
KCCM13320P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on January. 10. 2023 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 10. 2023.
¹ Where Rule 6.4(d) applies, such date is the date on which the status of international depositary authority was
acquired.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
Corynebacterium [?]es CJG0497 | Accession number given by the
INTERNATIONAL DEPOSITARY AUTHORITY:
KCCM13320P

II. SCIENTIFIC DESCRIPTION AND/OR PROPOSED TAXONOMIC DESIGNATION

The microorganism identified under I above was accompanied by:
☐ a scientific description
☐ a proposed taxonomic designation
(Mark with a cross where applicable)

III. RECEIPT AND ACCEPTANCE

This International Depositary Authority accepts the microorganism identified under I above,
which was received by it on January. 10. 2023 (date of the original deposit).¹

IV. RECEIPT OF REQUEST FOR CONVERSION

The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY

Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: January. 10. 2023 한국미생물보존센터장의인

¹ Where Rule 6.4(d) applies, such date is the date on which the status of international depositary authority was
acquired.

Form BP/4 (sole page)

한국미생물보존센터 사단법인 한국종균협회
03641 서울특별시 서대문구 홍제내2가길 45 유림빌딩 Tel. 02-391-0950, 396-0950 Fax. 02-392-2859

KOREAN CULTURE CENTER OF MICROORGANISMS KOREAN FEDERATION OF CULTURE COLLECTIONS
Yurim Bldg. 45, Hongjenae 2ga-gil, Seodaemun-gu, Seoul, 03641 Korea Tel: 82-2-391-0950, 396-0950 Fax: 82-2-392-2859

## Claims

1. A polypeptide having glutamine-hydrolyzing GMP synthase activity, comprising an amino acid sequence having 90% or more sequence identity to the amino acid sequence of SEQ ID NO: 1, the amino acid corresponding to the 123rd residue of the amino acid sequence of SEQ ID NO: 1 is substituted with another amino acid, the amino acid corresponding to the 441st residue is substituted with another amino acid, or a combination thereof.

2. The polypeptide according to claim 1, wherein the amino acid corresponding to the 123rd residue of the amino acid sequence of SEQ ID NO: 1 is substituted with lysine, glutamic acid, histidine, methionine, glutamine, arginine, serine, or tyrosine.

3. The polypeptide according to claim 1, wherein the amino acid corresponding to the 441st residue of the amino acid sequence of SEQ ID NO: 1 is substituted with valine, alanine, cysteine, glutamic acid, phenylalanine, histidine, isoleucine, lysine, leucine, asparagine, glutamine, threonine, or tryptophan.

4. The polypeptide according to claim 1, wherein the amino acid corresponding to the 123rd residue of the amino acid sequence of SEQ ID NO: 96 is substituted with lysine, glutamic acid, histidine, methionine, glutamine, arginine, serine, or tyrosine.

5. The polypeptide according to claim 1, wherein the amino acid corresponding to the 441st residue of the amino acid sequence of SEQ ID NO: 96 is substituted with valine, alanine, cysteine, glutamic acid, phenylalanine, histidine, isoleucine, lysine, leucine, asparagine, glutamine, threonine, or tryptophan.

6. The polypeptide according to claim 1, wherein the polypeptide comprises an amino acid sequence of any one SEQ ID NO selected from SEQ ID NOs: 52 to SEQ ID NO: 73 and SEQ ID NOs: 106 to 108.

7. A polynucleotide encoding the polypeptide of any one claim of claim 1 to claim 6.

8. The polynucleotide according to claim 7, wherein the polynucleotide comprises a nucleic acid sequence of any one SEQ ID NO selected from SEQ ID NOs: 74 to SEQ ID NO: 95 and SEQ ID NOs: 109 to 111.

9. A recombinant vector comprising the polynucleotide of claim 7.

10. A microorganism, comprising at least one selected from the group consisting of the polypeptide of any one claim of claim 1 to claim 6, a polynucleotide encoding the polypeptide, and a vector comprising the polynucleotide.

11. The microorganism according to claim 10, wherein the microorganism has increased producing ability of 5'-guanosine monophosphate (GMP).

12. The microorganism according to claim 10, wherein the microorganism is a microorganism of the genus *Corynebacterium*.

13. The microorganism according to claim 12, wherein the microorganism is *Corynebacterium stationis* o*r Corynebacterium casei*.

14. A method for producing 5'-guanosine monophosphate, comprising culturing a microorganism comprising at least one selected from the group consisting of the polypeptide of any one claim of claim 1 to claim 6, a polynucleotide encoding the polypeptide and a vector comprising the polynucleotide, in a medium.

15. The method for producing 5'-guanosine monophosphate according to claim 14, further comprising recovering 5'-guanosine monophosphate from the cultured microorganism, medium, or both.

16. The method for producing 5'-guanosine monophosphate according to claim 14, further comprising the step of culturing a microorganism producing 5'-xanthic acid (XMP), or the step of adding XMP to the medium, prior to the step of culturing the microorganism in a medium.

17. A composition for producing 5'-guanosine monophosphate, comprising a microorganism comprising at least one selected from the group consisting of the polypeptide of any one claim of claim 1 to claim 6, a polynucleotide encoding the polypeptide and a vector comprising the polynucleotide
